(19) Europäisches Patentamt / European Patent Office / Office européen des brevets

(11) **EP 3 856 277 B1**

(12) **EUROPÄISCHE PATENTSCHRIFT**

(45) Veröffentlichungstag und Bekanntmachung des Hinweises auf die Patenterteilung:
**17.06.2026  Patentblatt 2026/25**

(21) Anmeldenummer: **19779835.8**

(22) Anmeldetag: **26.09.2019**

(51) Internationale Patentklassifikation (IPC):
*A61M 60/187* (2021.01)   *A61M 60/289* (2021.01)
*A61M 60/468* (2021.01)   *A61M 60/47* (2021.01)
*A61M 60/495* (2021.01)   *A61M 60/861* (2021.01)
*A61M 60/863* (2021.01)   *A61M 60/865* (2021.01)

(52) Gemeinsame Patentklassifikation (CPC):
A61M 60/865; A61M 60/187; A61M 60/289;
A61M 60/468; A61M 60/47; A61M 60/495;
A61M 60/861; A61M 60/863

(86) Internationale Anmeldenummer:
PCT/EP2019/076017

(87) Internationale Veröffentlichungsnummer:
WO 2020/064922 (02.04.2020 Gazette 2020/14)

(54) **VORRICHTUNG, KIT ZUR UNTERSTÜTZUNG DER HERZAKTION UND EINFÜHRSYSTEM**

APPARATUS, KIT FOR SUPPORTING THE HEART ACTION AND INSERTION SYSTEM

DISPOSITIF, KIT POUR L'AIDE À L'ACTIVITÉ CARDIAQUE ET SYSTÈME D'INSERTION

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität:  28.09.2018  DE 102018124048

(43) Veröffentlichungstag der Anmeldung:
04.08.2021  Patentblatt 2021/31

(73) Patentinhaber: **Artract Medical Inc.**
New York  NY 10175 (US)

(72) Erfinder: **GOETZ, Wolfgang**
93051 Regensburg (DE)

(74) Vertreter: **Bobbert & Partner**
**Patentanwälte PartmbB**
**Postfach 1252**
**85422 Erding (DE)**

(56) Entgegenhaltungen:
WO-A1-2011/119100      WO-A1-2016/102561
WO-A1-2016/133203      US-A1- 2006 178 550

**Beschreibung**

Die Erfindung bezieht sich auf eine Vorrichtung zur Unterstützung der Herzaktion. Die Erfindung bezieht sich zudem auf ein Einführsystem und ein Kit zur Unterstützung der Herzaktion.

Zur Versorgung von Patienten mit Herzinsuffizienz sind unterschiedliche Verfahren und Systeme zur Herzunterstützung bzw. zur Unterstützung der Herzaktion bekannt. Beispielsweise gibt es linksventrikuläre und/oder rechtsventrikuläre Herzunterstützungssysteme, die pneumatisch, hydraulisch oder elektrisch angetrieben werden können. Die Systeme können vollständig implantiert werden, wobei die Energieversorgung beispielsweise telemetrisch oder kabelgestützt erfolgen kann.

Aus der WO 2011/119100 A1 ist eine Einrichtung und ein Verfahren zur kontrollierten Unterstützung der Bewegung einer Mitralklappe bekannt.

In der WO 2016/102561 A1 ist eine Einrichtung, ein System und ein Verfahren für die Verankerung im Muskelgewebe offenbart.

Aus der US 2006/0178550 A1 ist ein ventrikuläres Unterstützungs- und Hilfssystem bekannt.

In der WO 2016/133203 A1 ist ein ventrikuläres Unterstützungssystem mit einem internen implantierbaren Pumpsystem offenbart.

Es ist Aufgabe der vorliegenden Erfindung eine weitere Vorrichtung zur Unterstützung der Herzaktion bereitzustellen.

Darüber hinaus ist es Aufgabe, ein weiteres Einführsystem und ein weiteres Kit bereitzustellen.

Die erfindungsgemäße Aufgabe wird gelöst durch die Vorrichtung des Anspruchs 1, durch das Einführsystem des Anspruchs 11 sowie durch das Kit des Anspruchs 12.

Die erfindungsgemäße Aufgabe wird durch eine Vorrichtung zur Unterstützung der Herzaktion, vorzugsweise durch Verlagern der Herzbasis und/oder der Aortenwurzel, umfassend wenigstens einen ersten Anker und eine Zug- oder Führungseinrichtung zum Bewegen des ersten Ankers und/oder umfassend mindestens einen Hubantrieb, gelöst.

Die erfindungsgemäße Vorrichtung ist dazu ausgestaltet, die Herzbasis und/oder die Aortenwurzel zumindest zeitweise zu verlagern.

Zudem ist hierin eine Steuereinrichtung zum Antreiben einer Zug- oder Führungseinrichtung der erfindungsgemäßen Vorrichtung offenbart.

Die Erfindung betrifft ferner ein Einführsystem für die Vorrichtung. Das Einführsystem dient der Implantation der Vorrichtung in den Patienten. Das Einführsystem umfasst einen oder mehrere der folgenden Bestandteile in beliebiger Kombination: Einführungskatheter, Führungskatheter, Führungsdraht und Abgabekatheter. Dabei kann das Einführsystem in einigen Ausführungsformen auch mehrere Exemplare der jeweiligen Katheter und/oder des genannten Führungsdrahts umfassen.

Die Erfindung betrifft zudem ein Kit, das eine erfindungsgemäße Vorrichtung zur Unterstützung der Herzaktion, eine Steuereinrichtung, umfassend ein mechanisches, hydraulisches, pneumatisches, elektrisches oder magnetisches Antriebssystem zum Antreiben einer Zug- oder Führungseinrichtung der erfindungsgemäßen Vorrichtung, und/oder ein erfindungsgemäßes Einführsystem umfasst.

Zudem wird hierin ein Verfahren, insbesondere zur Unterstützung der Herzaktion, vorzugsweise durch Verlagerung der Herzbasis, des Herzskeletts und/oder der Aortenwurzel oder zum Unterstützen hierbei und/oder zum Implantieren einer erfindungsgemäßen Vorrichtung, offenbart. Das Verfahren umfasst die Schritte des Bereitstellens einer erfindungsgemäßen Vorrichtung und optional eines erfindungsgemäßen Einführsystems, des Implantierens eines ersten Ankers in oder an der Herzbasis, dem Herzskelett, der Aortenwurzel und/oder einer Struktur in örtlicher Nähe zur Aortenwurzel, insbesondere mittels des Einführsystems, des Implantierens der Zug- oder Führungseinrichtung zum Bewegen des ersten Ankers, insbesondere mittels des erfindungsgemäßen Einführsystems sowie des Verbindens des ersten Ankers und der Zug- oder Führungseinrichtung, insbesondere mittels des Einführsystems.

Das hierin offenbarte Verfahren ist insbesondere nicht zum Implantieren des ersten Ankers durch ein Lumen der Mitralklappe hindurch vorgesehen oder vorbereitet.

Das hierin offenbarte Verfahren ist insbesondere nicht zum Implantieren des ersten Ankers um einen Umfang oder um einen Teilumfang des Mitralklappenrings (Mitralklappenanulus) vorgesehen oder vorbereitet.

Das hierin offenbarte Verfahren kann weiterhin die Schritte des Implantierens eines zweiten Ankers in oder an der Herzspitze, einem Ligament, einer Rippe, einem Brustbein und/oder einer Struktur mit örtlicher Nähe zur Herzspitze sowie des Verbindens des ersten Ankers und des zweiten Ankers, insbesondere mittels des Einführsystems, umfassen.

Weiterhin kann das hierin offenbarte Verfahren die Schritte des Bereitstellens einer Steuereinheit, wie hierin offenbart, sowie des Bewegens der Zug- oder Führungseinrichtung unter Verwendung der Steuereinheit zur Unterstützung der Herzaktion umfassen.

Das sogenannte Herzskelett liegt anatomisch in unmittelbarer Umgebung der Aortenwurzel und kann kollagene Faserringe, Knorpelsubstanz, das rechte und linke Trigon sowie Herzklappenringe umfassen oder diese aufweisen.

Mithilfe des hierin offenbarten Verfahrens kann vorteilhaft die Herzaktion durch Verkürzung, die als Modulation bezeichnet werden kann, der langen Herzachse unterstützt werden.

Das Implantieren des ersten Ankers in oder an der Herzbasis, dem Herzskelett, der Aortenwurzel und/oder einer Struktur in örtlicher Nähe zur Aortenwurzel kann chirurgisch oder mittels des Einführsystems über eine Körpervene und über die Vena cava hin zum rechten Vorhof erfolgen. Das Implantieren des ersten Ankers kann chirurgisch, minimalinvasiv und/oder perkutan im Bereich der Herzbasis und/oder im Bereich des Vorhofseptums erfolgen.

Das Implantieren des zweiten Ankers kann chirurgisch, minimalinvasiv und/oder perkutan im Bereich der Herzspitze, in einer Rippe oder in der Nähe einer Rippe erfolgen.

Das Verbinden des ersten Ankers und des zweiten Ankers kann mittels eines aktiven, verkürzenden Elements erfolgen. Das aktive, verkürzende Element kann eine Zug- oder Führungseinrichtung sein.

Mithilfe des hierin offenbarten Verfahrens kann vorteilhaft die Strecke von der Herzbasis und/oder der Aortenwurzel zur Herzspitze aktiv verkürzt und so das Auswurfvolumen des Herzens vergrößert werden. Die Strecke kann ebenso die lange Achse des Herzens sein, die im Wesentlichen der Länge des Ventrikelseptums entspricht.

Mithilfe des hierin offenbarten Verfahrens kann vorteilhaft die Verlagerung bzw. die Streckung der Aortenwurzel (Aorta ascendens) während der Systole unterstützt werden. Dadurch kann der Hub der Aortenwurzel einschließlich einem Rückzug der Aortenwurzel in der Diastole vorteilhaft vergrößert werden.

Im Sinne der vorliegenden Erfindung kann ein Patient ein Mensch oder ein Tier sein. Ein Patient kann krank oder gesund sein.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlworte.

Soweit im Folgenden nicht anders ausgeführt, bedeutet proximal zur Körpermitte hin und distal von der Körpermitte weg.

Vorteilhafte Weiterentwicklungen der vorliegenden Erfindung sind jeweils Gegenstand von Unteransprüchen und Ausführungsformen.

Wenn hierin von einer Ausführungsform die Rede ist, so ist damit eine exemplarische, erfindungsgemäße Ausführungsform zu verstehen, die nicht als beschränkend zu verstehen ist.

Erfindungsgemäße Ausführungsformen können eines oder mehrere der oben und/oder im Folgenden genannten Merkmale in jeder beliebigen Kombination aufweisen, sofern eine solche nicht als technisch unmöglich für den Fachmann zu erkennen ist.

In einigen erfindungsgemäßen Ausführungsformen ist die Führungseinrichtung ein Hubantrieb oder weist einen solchen auf. Daher gelten die im Folgenden dargestellten und beschriebenen Ausführungen für den Hubantrieb in gleicher Weise für die Führungseinrichtung und umgekehrt.

In manchen erfindungsgemäßen Ausführungsformen ist die Zug- oder Führungseinrichtung vorzugsweise im Wesentlichen eine Linearführungseinrichtung oder weist eine solche auf.

In manchen erfindungsgemäßen Ausführungsformen weist der erste Anker einen V- oder U-förmigen Abschnitt auf. Alternativ ist der erste Anker V- oder U-förmig ausgebildet. Der erste Anker ist vorzugsweise mit wenigstens einer Linearführungsbuchse, weiter vorzugsweise mit zwei Linearführungsbuchsen verbunden. Eine Linearführungsbuchse kann mit einem Ende des ersten Ankers verbunden sein, zwei Linearführungsbuchsen können mit den beiden Enden des ersten Ankers verbunden sein.

Die Linearführungsbuchse ist zum Führen der Linearführungseinrichtung ausgestaltet.

In manchen erfindungsgemäßen Ausführungsformen ist die Linearführungseinrichtung ein Kolben oder weist eine solchen auf.

In manchen erfindungsgemäßen Ausführungsformen ist die Linearführungseinrichtung mechanisch, hydraulisch, pneumatisch, elektrisch oder magnetisch bewegbar. Sie kann hierfür entsprechend ausgestaltet sein. Ein entsprechender Antrieb oder Anschluss für einen solchen Antrieb kann vorgesehen sein.

In manchen erfindungsgemäßen Ausführungsformen ist eine Zugfeder in wenigstens einer der Linearführungsbuchsen zwischen der Linearführungseinrichtung und dem ersten Anker angeordnet.

In manchen erfindungsgemäßen Ausführungsformen ist die Verbindung zwischen dem ersten Anker und der Linearführungsbuchse eine Steckverbindung, eine Klemmverbindung, ein Bajonettverschluss oder eine andere Verbindung oder weist eine solche auf.

In manchen erfindungsgemäßen Ausführungsformen ist die Zug- oder Führungseinrichtung ein längliches, biegeweiches und/oder zugfestes Element, insbesondere ein Seil oder ein Riemen, oder weist ein solches auf.

In manchen erfindungsgemäßen Ausführungsformen ist die Vorrichtung aus einem Metall, einem Kunststoff und/oder einem Verbundwerkstoff hergestellt oder weist ein solches Material auf. Das Metall, der Kunststoff und/oder der Verbundwerkstoff ist vorzugsweise biokompatibel.

Ein Hubantrieb weist in manchen Ausführungsformen vorzugsweise einen längeren und einen kürzeren Betriebszustand auf. Dabei kann der Hubantrieb vorzugsweise von dem längeren Betriebszustand in den kürzeren und wieder zurück in den längeren Betriebszustand überführt werden, dies geschieht vorzugsweise periodisch, besonders vorzugsweise synchron zur Herzaktion des Patienten.

Der Hubantrieb als optionaler Teil der erfindungsgemäßen Vorrichtung kann vorzugsweise derart in einem Herz platziert werden, dass eine veränderliche Kraft auf einen Teil des Herzens ausgeübt wird. Beispielsweise kann der Hubantrieb dazu verwendet werden, Kraft auf die Herzbasis auszuüben, wobei die Kraft vorzugsweise zur Herzspitze hin gerichtet ist.

In einigen Ausführungsformen werden in der erfindungsgemäßen Vorrichtung zwei oder mehr Hubantriebe verwendet. Dabei kann beispielsweise vorgesehen sein, einen Hubantrieb in die linke und einen weiteren Hubantrieb in die rechte Herzkammer einzubringen. Dabei penetriert vorzugsweise einer der beiden Hubantriebe ausgehend vom rechten Ventrikel mit seinem ersten Ende die Trikuspidalklappe, vorzugsweise unmittelbar an der Basis des Klappensegels. Mit seinem zweiten Ende wird oder ist der Hubantrieb an der Herzspitze befestigt oder penetriert diese, und er ist beispielsweise am Sternum, am sterno-perikardialen Ligament oder an einer Rippe befestigt, beispielsweise über einen Anker.

Der andere der beiden Hubantriebe wird in einigen Ausführungsformen im linken Ventrikel platziert und penetriert mit seinem ersten Ende die Mitralklappe, vorzugsweise am Übergang von ihrem anterioren zu ihrem posterioren Segel, insbesondere in der Nähe der Kommissur zwischen anteriorem und posteriorem Segel, besonders vorzugsweise unmittelbar an der Basis des Klappensegels. Das zweite Ende des im linken Ventrikel platzierten Hubantriebs ist an der Herzspitze befestigt oder penetriert diese und ist beispielsweise am Sternum, am sterno-perikardialen Ligament oder an einer Rippe befestigt, beispielsweise über einen zweiten Anker.

In einigen Ausführungsformen sind die Hubantriebe an ihren ersten Enden über den ersten Anker, der als Verbindungsstück bezeichnet werden kann, direkt oder indirekt miteinander verbunden. Dazu befindet sich das Verbindungsstück nach der Implantation vorzugsweise in den Vorhöfen des Herzens und penetriert dabei das Vorhofseptum.

Vorzugsweise sind die Hubantriebe derart im Herzen befestigt, dass eine Verkürzung der Hubantriebe zu einer Unterstützung der Herzaktion führt, insbesondere durch Verlagerung der Herzbasis und/oder der Aortenwurzel. Eine Veränderung der Länge der Hubantriebe in Form einer Verkürzung oder Verlängerung kann ein Verschieben eines Kolbens relativ zu einem Zylinder der Hubantriebe bedeuten. Wenn beispielsweise der Kolben in den Zylinder des Hubantriebs hineingeschoben wird, verkürzt sich die Gesamtlänge (Verkürzung des Hubantriebs), bei einem Herausziehen oder Herausführen des Kolbens aus dem Zylinder verlängert sich die Gesamtlänge (Verlängerung des Hubantriebs). Dies gilt analog für die Führungseinrichtung. Ein Hineinschieben oder Hineinziehen einer Linearführungseinrichtung in eine Linearführungsbuchse führt zu einer Verkürzung der Führungseinrichtung, ein Herausführen oder Herausziehen der Linearführungseinrichtung aus der Linearführungsbuchse führt zu einer Verlängerung der Führungseinrichtung.

Die erfindungsgemäße Vorrichtung kann in einigen Ausführungsformen vollständig chirurgisch über eine offene Herzoperation implantiert werden und entsprechend ausgestaltet sein. In einigen Fällen kann die Vorrichtung über einen Katheterzugang eingesetzt werden. Alternativ ist auch eine Kombination von offener Herzoperation und kathetergestütztem Verfahren möglich.

Die Verkürzung des oder der Hubantriebe kann in einigen Ausführungsformen hydraulisch, pneumatisch und/oder magnetisch (beispielsweise durch einen Linearmotor oder einen Linearaktor) geschehen. Dazu können die Hubantriebe über einen eigenen Antrieb verfügen. Alternativ kann den Hubantrieben extern eine Kraft zu ihrer Verkürzung zugeführt werden, beispielsweise aus einer separat angeordneten Steuereinheit.

In einigen Ausführungsformen sind innerhalb der Hubantriebe und/oder innerhalb des Verbindungsstücks ein oder mehrere Seile vorhanden, die vorzugsweise zur Kraftübertragung auf die Hubantriebe zu deren Verkürzung eingesetzt werden können. Vorzugsweise ist ein Seil innerhalb der Hubantriebe als ein Seilzug angeordnet. Dabei ist vorzugsweise ein Ende des Seils an einem zweiten Anker an der Herzspitze, dem sterno-perikardialen Ligament oder an einer Rippe befestigt oder hierzu vorgesehen, wobei das andere Ende vorzugsweise frei ist und durch Zug an dem freien Seilende beide Hubantriebe (welche endseitig abgestützt sein können) verkürzt werden können.

In einigen Ausführungsformen umfasst jeder Hubantrieb einen Kolben und einen Zylinder, wobei sich bei einer Verkürzung des Hubantriebs der Kolben in den Zylinder schieben kann. Verkürzung und Verlängerung der Hubantriebe kann dabei beispielsweise durch eine Druckänderung einer, in den Zylindern enthaltenen, Flüssigkeit verursacht werden. Die Flüssigkeit kann dabei beispielsweise aus einer internen oder externen Steuereinheit zugeführt werden.

In einigen Ausführungsformen sind in die Zylinder elastische Elemente, beispielsweise Druck- oder Zugfedern, eingebracht, die beispielsweise einer Verkürzung beziehungsweise einer Verlängerung der Hubantriebe elastisch entgegenwirken.

Die Steuereinheit (hierin auch als Steuereinrichtung bezeichnet) kann über eine Energiequelle verfügen und/oder mit einer solchen verbunden sein. Die Steuereinheit kann ferner über Sensoren verfügen, die es ermöglichen oder dazu beitragen, die Vorrichtung synchron zur Herzaktion anzusteuern.

In einigen Ausführungsformen umfasst die erfindungsgemäße Vorrichtung mindestens einen Hubantrieb, der die Herzbasis und/oder die Aortenwurzel während der Herzaktion verlagert. Eine Verlagerung kann ein Verschieben, insbesondere ein translatorisches Verschieben bedeuten. Dadurch wird vorzugsweise in einem Teil des Herzzyklus die lange Achse des Herzens verkürzt und die Aortenwurzel gestreckt. Vorzugsweise wird der Hubantrieb anhand von Sensordaten synchron zur Herzaktion bewegt.

Vorzugsweise ist die Vorrichtung zur zumindest abschnittsweisen Implantation in ein Herz ausgestaltet. Die Vorrichtung ist vorzugsweise derart ausgestaltet, dass sie zumindest teilweise in ein menschliches Herz implantiert werden kann. In einigen Ausführungsformen ist die Vorrichtung derart ausgestaltet, dass sie an einem Abschnitt mit einer Rippe und/oder dem Brustbein und in einem weiteren Abschnitt mit dem Herz, insbesondere der Herzbasis verbunden werden kann.

In einigen Ausführungsformen der erfindungsgemäßen Vorrichtung ist diese derart gestaltet, dass sich mehrere dieser Vorrichtungen zur gleichen Zeit im Herzen befinden können und die Herzaktion unterstützen. In diesem Fall werden vorzugsweise mehrere der Vorrichtungen in einem Herz implantiert.

Vorzugsweise befinden sich in einigen Ausführungsformen nach der Implantation der Vorrichtung ein erster Abschnitt der Vorrichtung im linken Ventrikel und ein weiterer Abschnitt im rechten Ventrikel. In einigen Ausführungsformen befinden sich eine Vorrichtung zumindest teilweise im linken Ventrikel und eine weitere Vorrichtung zumindest teilweise im rechten Ventrikel. Dabei sind die Vorrichtungen vorzugsweise derart gestaltet, dass sie nach dem Implantieren in das Herz die Herzbasis mit der Herzspitze verbinden können, wobei die Vorrichtungen jeweils in der Länge veränderbar sind.

In einigen Ausführungsformen ist der erste Anker derart ausgestaltet, dass bei einer Verkürzung der Vorrichtung nach der Implantation das Herzskelett, die Herzbasis und/oder die Aortenwurzel in Richtung Herzspitze gezogen wird bzw. werden. Dabei wird beispielsweise gespeicherte Energie, z. B. Federenergie oder elektrische Energie, in kinetische Energie, d. h. Bewegungsenergie, umgesetzt.

In einigen Ausführungsformen weist die Vorrichtung einen zweiten Anker zur Implantation in oder an der Herzspitze, einem Ligament, einer Rippe, einem Brustbein und/oder einer Struktur mit örtlicher Nähe zur Herzspitze auf.

Erfindungsgemäß weist der erste Anker zur Implantation in oder an der Herzbasis einen Bügel auf oder ist ein solcher, der derart implantiert werden kann, dass er sich vom linken in das rechte Atrium erstreckt und dabei z. B. das untere Ende des interatrialen Septums zwischen Mitralklappenanulus und Trikuspidalklappenanulus durchdringt. Der Bügel kann beispielsweise ein Abschnitt des ersten Ankers sein.

Der Bügel liegt nach der Implantation vorzugsweise über dem Herzskelett, insbesondere über dem rechten Trigon.

Während des gesamten Herzzyklus verändert sich vorzugsweise die Lage des zweiten Ankers nach der Implantation nicht oder nicht wesentlich. Bei Verkürzung der Vorrichtung werden so das Herzskelett, die Herzbasis und/oder die Aortenwurzel in Richtung der Herzspitze gezogen.

In einigen Ausführungsformen weist die Vorrichtung mehr als zwei Anker, insbesondere drei, vier oder sechs Anker auf.

In einigen Ausführungsformen ist die Vorrichtung ausgebildet, um sich während der Systole zu verkürzen und während der Diastole zu verlängern.

Vorzugsweise wird die Vorrichtung in einer Länge verwendet, die der Länge des Herzens und/oder der Körpergröße des Patienten angepasst ist.

Vorzugsweise weist die Vorrichtung in der Diastole eine Länge zwischen 4cm und 20cm, besonders vorzugsweise zwischen 7cm und 16cm auf.

Vorzugsweise weist die Vorrichtung in der Systole eine Länge zwischen 4cm und 16cm, besonders vorzugsweise zwischen 6cm und 14cm auf.

Vorzugsweise verkürzt sich die Vorrichtung in der Systole gegenüber der Diastole um zwischen 3% und 40%, besonders vorzugsweise zwischen 5% und 20%.

Die genannten Angaben zu Längen und Verkürzung beziehen sich vorzugsweise auf den Abschnitt der Vorrichtung, der sich zwischen dem ersten und dem zweiten Anker befindet.

Die Vorrichtung unterstützt vorzugsweise die natürliche Bewegung der Herzbasis, des Herzskeletts und/oder der Aortenwurzel und vergrößert die Amplitude dieser Bewegung, wodurch die Dehnung der Aortenwurzel in der Systole und der daraus resultierende elastische Rückzug der Aortenwurzel in der Diastole verstärkt wird, wodurch wiederum die diastolische Pumpwirkung der aufsteigenden Aorta unterstützt wird.

In einigen Ausführungsformen ist der erste Anker ein Implantat, insbesondere ein intravaskulärer Stent, zur Platzierung in der Aorta oder eine Aortenklappenprothese.

In einigen Ausführungsformen ist die Energie, die die Vorrichtung zur Unterstützung der Herzaktion verwendet, Spannungsenergie eines elastischen Elements der Vorrichtung.

Die Spannungsenergie wird in diesem Fall vorzugsweise durch ein elastisches Element, insbesondere durch eine Feder, übertragen. Die Kraft des elastischen Elements gleicht vorzugsweise die elastische Rückstellkraft der Aortenwurzel aus.

In einigen Ausführungsformen wird die Kraft, die der Vorrichtung zur Unterstützung der Herzaktion zugeführt wird, mechanisch oder magnetisch von einer Energiequelle über eine Verbindungseinheit übertragen. Die Vorrichtung weist vorzugsweise eine Verbindungseinheit zum Übertragen von Kraft auf den im Herz implantierten Teil der Vorrichtung auf. Die Kraftübertragung kann beispielsweise über einen Bowdenzug, pneumatisch, hydraulisch oder magnetisch erfolgen.

Die Vorrichtung weist vorzugsweise eine Energiequelle auf oder ist mit einer solchen verbunden. Die Energiequelle kann in einigen Ausführungsformen eine Kraft bereitstellen, die auf den Teil der Vorrichtung, der sich in der Systole verkürzt, übertragen wird. Dazu weist die Energiequelle ein Element auf, das eine gespeicherte Energie in Bewegungsenergie umsetzen kann, beispielsweise einen Linearaktuator oder einen elektrischen Motor, der als rotierender Motor oder als Linearmotor ausgeführt sein kann. Die Energiequelle kann alternativ oder zusätzlich über einen Kompressor zur Bereitstellung eines Fluids unter Druck verfügen. Als Energiespeicher kann beispielsweise eine Batterie verwendet werden.

In einigen Ausführungsformen enthält die Vorrichtung eine Einheit zur Wandlung von übertragener Energie aus einer separaten Energiequelle. In einigen Ausführungsformen wird der Vorrichtung Energie in Form von elektrischer Energie bereitgestellt. Die Vorrichtung verfügt vorzugsweise über ein Element, das die elektrische Energie in Bewegungsenergie umwandeln kann, beispielsweise einen elektrischen, rotierenden Motor oder Linearmotor oder einen Linearaktuator.

In einigen Ausführungsformen ist die Kraft, die von der Vorrichtung zur Unterstützung der Herzaktion verwendet wird, eine pneumatische oder hydraulische Kraft. Dazu wird in einem Teil der Vorrichtung oder außerhalb der Vorrichtung ein Fluid mit Druck beaufschlagt, wobei das Fluid vorzugsweise in Kommunikation mit einem beweglichen Teil der Vorrichtung steht. Der Druck des Fluids ist dabei vorzugsweise über eine Herzaktion variabel. Die Vorrichtung weist vorzugsweise einen hydraulischen beziehungsweise pneumatischen Zylinder auf zur Umsetzung des Drucks in eine Kraft zur Verkürzung und/oder Verlängerung der Vorrichtung.

Die pneumatische Kraft oder hydraulische Kraft wird in der Vorrichtung und/oder außerhalb der Vorrichtung erzeugt. In einigen Ausführungsformen wird elektrische Energie über einen elektrischen Leiter zu der Vorrichtung übertragen und dort in pneumatische und/oder hydraulische Energie umgewandelt.

In einigen Ausführungsformen umfasst die Vorrichtung eine Energiequelle, die die zur Unterstützung der Herzaktion benötigte Energie als elektrische, pneumatische, hydraulische oder magnetische Energie bereitstellt.

Die genannten Energieformen werden vorzugsweise zur Verkürzung und/oder Verlängerung von Teilen der Vorrichtung eingesetzt. In einigen Ausführungsformen wird die Energiequelle mit Nuklearenergie betrieben.

In einigen Ausführungsformen ist die Vorrichtung zwischen einer diastolischen Aufwärtsposition und einer systolischen Abwärtsposition bistabil. Dies bedeutet, dass die Vorrichtung vorzugsweise in einer diastolischen und/oder einer systolischen Endposition stabil angeordnet bzw. positioniert ist, sich also vorzugsweise nicht aus dieser Position ohne äußeres Zutun herausbewegt.

In einigen Ausführungsformen weist die Vorrichtung einen Gleichgewichtszustand in ihrer Aufwärts- bzw. Abwärtsposition (oder oberen oder unteren Position) auf, wobei sich vorzugsweise die Vorrichtung im Betrieb zwischen den beiden stabilen Positionen bewegt, wenn der Vorrichtung Energie aus einer der separaten Energiequellen steuerbar zugeführt wird.

In einigen Ausführungsformen umfasst die erfindungsgemäße Vorrichtung oder das erfindungsgemäße Kit eine Steuereinrichtung. Eine Steuereinrichtung ist hierin auch als Steuereinheit bezeichnet, und umgekehrt. Die Steuereinheit ist dabei vorzugsweise konfiguriert, um die Vorrichtung abhängig vom Herzzyklus zu steuern. Die Steuereinheit umfasst vorzugsweise eine Recheneinheit.

In einigen Ausführungsformen umfasst die Steuereinheit Sensoren oder ist mit Sensoren verbunden. Die Sensoren sind dabei vorzugsweise dazu geeignet, physiologische Parameter zu messen, die mit der Herzaktivität in Verbindung stehen. Die Sensoren sind dabei vorzugsweise derart ausgestaltet, dass sie ein Sensorsignal bereitstellen, das der Steuereinheit zur Verfügung gestellt wird. Die Steuereinheit ist dabei konfiguriert, um die Verkürzung und/oder Zugkraft der Vorrichtung zu steuern oder zu regeln, vorzugsweise abhängig von Sensorsignalen.

In einigen Ausführungsformen steuert die Steuereinheit die Frequenz, Geschwindigkeit, Pausen und/oder die Kraft der wiederholenden Verkürzung und Verlängerung der Vorrichtung.

In einigen Ausführungsformen umfasst die Steuereinrichtung Sensoren, die die elektrische Herzaktivität messen, oder ist mit solchen Sensoren verbunden. Die Sensoren umfassen in einigen Ausführungsformen Drucksensoren (die beispielsweise den Blutdruck in Gefäßen und/oder in einer oder mehreren Herzkammern messen), Sensoren für Kraft, elektrische Spannung und/oder elektrischen Strom und/oder Sensoren für das Ausmaß der Verkürzung der Vorrichtung. Die Steuereinrichtung nimmt in einigen Ausführungsformen ein EKG-Signal oder ein anderes Signal auf, um die Vorrichtung synchron zur Herzaktion zu steuern.

In einigen Ausführungsformen kann die erfindungsgemäße Vorrichtung mit Hilfe von Herzchirurgie, minimalinvasiver Chirurgie oder mit Hilfe von perkutanem Katheter implantiert werden, wobei die Vorrichtung nach Implantation vorzugsweise die Aortenwurzel mit der Herzspitze und/oder mit der Brustwand und/oder mit einer Rippe verbindet. Die Verkürzung der erfindungsgemäßen Vorrichtung führt vorzugsweise zu einem Zug an der Aortenwurzel und vergrößert damit den Hub der Aortenwurzel während des Herzzyklus. Der vergrößerte Hub der Aortenwurzel vergrößert in der Regel die Rückstellkraft der Aortenwurzel, was in einigen Fällen zu einem vergrößerten Rückzug der Aortenwurzel in der Diastole und damit verbundenen vergrößerten Pumpvolumen der Aortenwurzel in der Diastole führt.

Die erfindungsgemäße Vorrichtung ist in einigen Ausführungsformen an dem fibrinösen Skelett zwischen Mitralklappenring und Trikuspidalklappenring verankert. Die Vorrichtung vergrößert vorzugsweise den Hub des Herzskelettes und insbesondere der damit verbundenen gesamten Herzbasis synchron zum Herzzyklus.

Die erfindungsgemäße Vorrichtung hat vorzugsweise keinen direkten Einfluss auf die Herzmuskelkontraktion und auf die vom Zentrum der Herzbasis entfernten, nicht mit dem fibrinösen Skelett des Herzens verbundenen Anteile des Herzens wie z. B. die von der Aortenwurzel entfernten Anteile des Mitral- und Trikuspidalklappenrings oder den venösen Koronarsinus, bei denen kein oder nur ein rudimentäres Herzskelett existiert.

Die Aortenwurzel ist mit der Herzbasis und dem fibrinösen Skelett des Herzens verbunden. Die Herzbasis ist eine Struktur, in der die vier Herzklappen in einer Ebene liegen. Die Herzbasis beinhaltet das Herzskelett, das eine Struktur aus knorpeligem Gewebe ist, die den Aortenklappenring, den Mitralklappenring und den Trikuspidalklappenring einschließt.

Wenn sich das Herz in der Systole kontrahiert, verringert sich der Durchmesser der Herzkammern. Zudem verkürzen sich auch die Herzkammern in Längsrichtung. Während der Systole bewegen sich das Herzskelett und die Herzbasis mit den vier Herzklappen mit der Aortenwurzel synchron in Richtung der Herzspitze. Dabei wird die Aortenwurzel gestreckt und elastisch gedehnt. Während der Diastole wird das Herzskelett mit der Herzbasis von der elastischen Aortenwurzel wieder in die entgegengesetzte Richtung, weg von der Herzspitze, gezogen. Während des gesamten Herzzyklus bleibt die Herzspitze hingegen annähernd stationär, was unter anderem durch das Perikard verursacht wird. Das Perikard ist ein geschlossener Sack, der circa 10ml Flüssigkeit enthält und das gesamte Herz umfasst. Das geschlossene Perikard ist flüssigkeitsdicht und erlaubt somit keinen Flüssigkeitsaustausch mit den umgebenden Körperhöhlen. Bei der Kontraktion des Herzens kann das zum Teil frei im Brustkorb liegende Perikard der Verringerung des Herzumfangs folgen. Das Perikard ist jedoch im Bereich der Herzspitze über das sterno-perikardiale Ligament mit dem Manubrium sterni und dem Processus xiphoideus verbunden. Bei der Kontraktion des Herzens können sich das Perikard und die in dem geschlossenen Perikard liegende Herzspitze nicht von der Brustwand hin zur Herzbasis bewegen. Aus diesem Grund führen die Kontraktion des Herzmuskels und die Verkürzung des Herzens zu einem Zug an der Herzbasis und der elastischen Aortenwurzel, die auf diese Weise in der Systole gestreckt wird. Sobald sich der Herzmuskel in der Diastole entspannt, zieht die gestreckte Aortenwurzel durch die in ihr gespeicherte Spannungsenergie die Herzbasis weg von der stationären Herzspitze. Bei Untersuchungen mit Schafen, die in Bezug auf das Herz eine mit dem Menschen vergleichbare Anatomie aufweisen, zeigt sich, dass in Ruhe durch die Spannung eine Kraft zwischen Aortenwurzel und Herzbasis von 1,8 $\pm$0,2N besteht. Um die Aortenwurzel 10 mm in Richtung der Herzspitze zu bewegen, muss eine Kraft F von 1,8 $\pm$0,1N aufgewendet werden. Der normale Ausschlag des Hubs (s) der Aortenwurzel beträgt beim gesunden Menschen 12$\pm$2 mm. Pro Herzschlag wird damit zur Auslenkung der Aortenwurzel eine Arbeit W von etwa

$$W = F * s = 1,8\,N * 0,012\,m = 0,0216\,J$$

verrichtet, wobei die entsprechende Energie zum Teil in der elastischen Dehnung der Aortenwurzel gespeichert wird.

Bei der Dehnung und Verlängerung der Aortenwurzel in der Systole wird somit ein Teil der Energie der Herzmuskelkontraktion durch die Spannung elastischer Fasern gespeichert. Bei der Entspannung des Herzmuskels und nach dem Schließen der Aortenklappe zu Beginn der Diastole ziehen sich die elastischen Fasern der Aortenwurzel wieder zusammen und die Aortenwurzel bewegt sich weg von der stationären Herzspitze und pumpt Blut in den Körperkreislauf. Dies ist ein wesentlicher Teil der Windkesselfunktion der Aortenwurzel. Bei einem Erwachsenen wird so etwa die Hälfte des Schlagvolumens in der Aortenwurzel zwischengespeichert, d.h. in der Diastole pumpt die Aortenwurzel zeitverzögert zur Systole das halbe Schlagvolumen vorwärts. Zusätzlich bewirkt der elastische Rückzug der Aortenwurzel in der Diastole einen Sog in der linken Herzkammer. Durch den Rückzug der Aortenwurzel während der Diastole wird Blut aus den Vorhöfen in die Herzkammern gesaugt, was einen kurzzeitigen Unterdruck zu Beginn der Diastole in beiden Herzkammern bis zu -100mmH$_2$O (-7, 3 mmHg) erklärt. Dieser Sog erhöht das Blutvolumen in den Herzkammern am Ende der Diastole bzw. die Vorlast und verbessert so aufgrund des Frank-Starling-Mechanismus den Auswurf des Herzens aufgrund der Verschiebung der Frank-Starling-Kurve nach rechts. Der Rückzug der Aortenwurzel während der Diastole verringert zudem den Druck in der Aortenwurzel zu Beginn der Systole und reduziert so die Nachlast des Herzens.

Herzschwäche, insbesondere Herzinsuffizienz, besteht, wenn das Herz nur noch weniger als 45% des Herzkammervolumens auswerfen kann. Von einer hochgradigen Einschränkung spricht man bei einer Auswurfleistung von weniger als 35%.

Zu der Symptomatik der Herzinsuffizienz zählt Atemnot (Belastungs-, Ruhedyspnoe, Orthopnoe, paroxysmale nächtliche Dyspnoe), Müdigkeit, inadäquate Erschöpfung nach Belastungen, Schwäche, Lethargie, Flüssigkeitsretention (Bein- oder Bauchschwellung, Gewichtszunahme), nächtliches Wasserlassen (Nykturie), trockener Husten (besonders nachts), Schwindel, Synkopen, Inappetenz, Übelkeit, Völlegefühl, Meteorismus, Obstipation, abdominelle Schmerzen, unter Umständen Gewichtsabnahme, Gedächtnisstörungen, Verwirrtheitszustände und kognitive Beeinträchtigungen.

Die Herzinsuffizienz ist nach Schweregrad eingeteilt in NYHA I (diagnostizierte Herzkrankheit ohne Symptome und ohne Einschränkung der Belastbarkeit), NYHA II (leichte Einschränkung der Belastbarkeit, keine Symptome in Ruhe, sondern erst bei stärkerer Belastung), NYHA III (starke Einschränkung der Belastbarkeit, keine Symptome in Ruhe, jedoch bereits bei leichter Belastung) und NYHA IV (persistierende Symptomatik auch in Ruhe). Bei der Diagnostik der Herzinsuffizienz ist vor allem die Echokardiographie von Bedeutung, mit der regionale und globale Einschränkungen der Herzfunktion beurteilt werden können.

Patienten mit einer Herzschwäche mit einer linksventrikulären Ejektionsfraktion (EF) von weniger als 45 % zeigen einen verringerten Hub der Aortenwurzel auf weniger als 8 mm während der Systole. Die Verkürzung der langen Herzachse ist ein besonders sensibler Parameter für die Messung der Herzfunktion. Bei diesen Patienten wird während der Systole die Aortenwurzel unzureichend in Richtung der Herzspitze bewegt und es wird keine ausreichende Spannenergie in der Aortenwurzel gespeichert. Während der Diastole steht so keine elastische Rückstellkraft zur Verfügung. Dadurch geht das Schlagvolumen in der Diastole verloren, die Nachlast des Herzens steigt und es wird in der Diastole kein Sog in die Herzkammern erzeugt.

Bei einer Herzinsuffizienz mit reduzierter Ejektionsfraktion (HFrEF) wird in der Echokardiographie eine verminderte Wandbewegung des Herzens mit verringerter Auswurffraktion von weniger als 45 % gemessen. Dabei zeigt sich, dass die Verkürzung sowohl der langen Achse von Herzspitze zu Herzbasis als auch der dazu orthogonal gelegenen kurzen Achse verringert ist. Mit zweidimensionaler Echokardiographie (Simpson-Methode) kann die Verringerung des Auswurfvolumens bestimmt werden.

Mindestens die Hälfte der Patienten mit Symptomen der Herzinsuffizienz hat eine in der üblichen Echokardiographie gemessene normale Herzfunktion mit einer normalen Verkürzung des orthogonalen Durchmessers der Herzkammern. Die daraus errechnete Ejektionsfraktion erscheint als normal. Daher wird diese Form der Herzinsuffizienz als Herzinsuffizienz mit erhaltener Ejektionsfraktion (HFpEF) bezeichnet. Als Ursache der Herzinsuffizienz werden eine Versteifung der Herzmuskulatur und eine beeinträchtigte diastolische Funktion des Herzens diskutiert. Bei genauerer echokardiographischer Betrachtung der Herzfunktion dieser Patienten zeigt sich häufig jedoch eine Veränderung der Ver- und Entwindung der linken Herzkammer sowie eine Verminderung des diastolischen Sogs und der frühen diastolischen Füllung der linken Herzkammer.

Die normale Bewegung der Aortenwurzel mit der Herzbasis hin zur Herzspitze von 12±2 mm ist bei der HFpEF auf deutlich weniger als 8 mm verringert. Die Geschwindigkeit des Hubes der Aortenwurzel in der Systole und frühen Diastole ist hochsignifikant vermindert mit 0,64±0,51cm/s im Vergleich zu 1,54±0,51cm/s in der Systole und 1,49±0,77cm/s im Vergleich zu 2,32±1,24cm/s zu Beginn der Diastole. Die mangelnde Leistungsfähigkeit und die Herzinsuffizienzsymptome dieser Patienten korrelieren mit einer geringeren Elastizität, insbesondere einer Versteifung der Aortenwurzel. Gleichzeitig findet sich bei diesen Patienten eine Verdickung des Herzmuskels, was auf eine Kompensation der geringeren Windkesselfunktion und einen geringeren Hub der Aortenwurzel hindeutet. Die Herzkraft erscheint in der Echokardiographie noch normal oder sogar erhöht, reicht jedoch nicht mehr aus, um die Aortenwurzel zur Herzspitze zu ziehen, da diese weniger elastisch ist und eine erhöhte Kraft erforderlich wäre, um einen ausreichenden Hub der Aortenwurzel zu bewirken.

Die erfindungsgemäße Vorrichtung soll bevorzugt verwendet werden bei chronischer Herzinsuffizienz mit erhaltener linksventrikulärer Ejektionsfraktion (HFpEF), bei der die systolische Herzmuskelfunktion weitgehend erhalten ist, jedoch die Herzkraft nicht mehr ausreicht, eine durch verschiedene Erkrankungen oder durch Alterungsprozesse versteifte Aortenwurzel zu dehnen und damit verbunden einen ausreichenden Hub der Herzbasis bzw. Streckung der Aortenwurzel zu erzeugen.

In einigen erfindungsgemäßen Ausführungsformen ist die Vorrichtung zur Unterstützung der Herzaktion durch Verlagerung der Herzbasis und/oder der Aortenwurzel vorgesehen und umfasst mindestens einen Hubantrieb.

In einigen erfindungsgemäßen Ausführungsformen ist die Vorrichtung zur zumindest abschnittsweisen Implantation in ein Herz ausgestaltet.

In einigen erfindungsgemäßen Ausführungsformen weist die Vorrichtung einen ersten Anker zur Implantation in oder an der Herzbasis, dem Herzskelett, der Aortenwurzel und/oder einer Struktur in örtlicher Nähe zur Aortenwurzel auf.

In einigen erfindungsgemäßen Ausführungsformen weist die Vorrichtung einen zweiten Anker zur Implantation in oder an der Herzspitze, einem Ligament, einer Rippe, einem Brustbein und/oder einer Struktur mit örtlicher Nähe zur Herzspitze auf.

In einigen erfindungsgemäßen Ausführungsformen weist der erste Anker zur Implantation in oder an der Herzbasis einen Bügel auf, der derart implantiert werden kann, dass er sich vom linken Atrium in das rechte Atrium erstreckt und das untere Ende des interatrialen Septums zwischen Mitralklappenanulus und Trikuspidalklappenanulus durchdringt.

In einigen erfindungsgemäßen Ausführungsformen ist die Vorrichtung ausgebildet, um sich während der Systole zu verkürzen und während der Diastole zu verlängern.

In einigen erfindungsgemäßen Ausführungsformen ist der erste Anker ein Implantat, insbesondere ein intravaskulärer Stent, in der Aorta oder eine Aortenklappenprothese.

In einigen erfindungsgemäßen Ausführungsformen ist die Energie, die die Vorrichtung zur Unterstützung der Herzaktion verwendet, Spannungsenergie eines elastischen Elements der Vorrichtung.

In einigen erfindungsgemäßen Ausführungsformen wird die Kraft, die der Vorrichtung zur Unterstützung der Herzaktion zugeführt wird, mechanisch oder magnetisch von einer Energiequelle über eine Verbindungseinheit übertragen.

In einigen erfindungsgemäßen Ausführungsformen ist die Kraft, die von der Vorrichtung zur Unterstützung der Herzaktion verwendet wird, eine pneumatische oder hydraulische Kraft.

In einigen erfindungsgemäßen Ausführungsformen umfasst die Vorrichtung eine Energiequelle, die die zur Unterstützung der Herzaktion benötigte Energie als elektrische, pneumatische, hydraulische oder magnetische Energie bereitstellt.

In einigen erfindungsgemäßen Ausführungsformen ist die Vorrichtung zwischen einer diastolischen Aufwärtsposition und einer systolischen Abwärtsposition bistabil.

In einigen erfindungsgemäßen Ausführungsformen umfasst die Vorrichtung eine Steuereinheit.

In einigen erfindungsgemäßen Ausführungsformen umfasst das Einführsystem einen Einführungskatheter, einen Führungskatheter, einen Führungsdraht und/oder mindestens einen Abgabekatheter.

In einigen Ausführungsformen umfasst ein Verfahren zur Steuerung einer erfindungsgemäßen Vorrichtung die Schritte Bereitstellen einer erfindungsgemäßen Vorrichtung mit einer Steuereinheit und Steuern der erfindungsgemäßen Vorrichtung unter Verwendung der Steuereinheit zur Unterstützung der Herzaktion.

In einigen erfindungsgemäßen Ausführungsformen ist ein digitales Speichermedium mit elektronisch auslesbaren Steuersignalen konfiguriert, um derart mit einer programmierbaren Recheneinheit zusammenzuwirken, dass die maschinellen Schritte des Verfahrens zur Steuerung einer erfindungsgemäßen Vorrichtung veranlasst werden.

In einigen erfindungsgemäßen Ausführungsformen wird ein Computerprogramm-Produkt, als Signalwelle oder mit einem auf einem maschinenlesbaren Träger gespeicherten Programmcode, zur Veranlassung der maschinellen Schritte des Verfahrens zur Steuerung einer erfindungsgemäßen Vorrichtung bereitgestellt, wenn das Computerprogramm-Produkt auf einer Recheneinheit abläuft.

In einigen erfindungsgemäßen Ausführungsformen wird ein Computerprogramm mit einem Programmcode zur Veranlassung der maschinellen Schritte des Verfahrens zur Steuerung einer erfindungsgemäßen Vorrichtung bereitgestellt, wenn das Computerprogramm auf einer Recheneinheit abläuft.

In einigen erfindungsgemäßen Ausführungsformen spannt das Verbindungsstück (auch: Anker) allein oder gemeinsam mit einer oder mehreren Führungseinrichtungen eine erste Haupterstreckungsebene auf. In bestimmten Ausführungsformen weist die Vorrichtung keine Struktur, insbesondere keine Erweiterung oder Erweiterungseinheit, auf, die in einer die erste Haupterstreckungsebene unter einem Winkel zwischen 70° und 110° schneidende zweiten Haupterstreckungsebene liegt.

In manchen erfindungsgemäßen Ausführungsformen ist/sind die Führungseinrichtung(en) der Vorrichtung nicht vorgesehen, um durch die (bereits vorhandenen) Klappenöffnungen des Herzens, insbesondere durch die Mitralklappenöffnung und/oder die Trikuspidalklappenöffnung, geführt, in diesen positioniert oder in diesen fixiert zu werden.

In einigen erfindungsgemäßen Ausführungsformen wird die Mitralklappe nicht hinter dem posterioren Mitralklappensegel von einer der Führungseinrichtungen penetriert.

Einige der erfindungsgemäßen Ausführungsformen können die folgenden Vorteile aufweisen:
Die erfindungsgemäße Vorrichtung unterstützt vorzugsweise die Dehnung und den Hub der Aortenwurzel.

Durch einen größeren Hub der Aortenwurzel wird die elastische Vordehnung der Aortenwurzel in der Systole erhöht und es entsteht während der Diastole ein größerer elastischer Rückzug der Aortenwurzel. Dadurch wird die Menge des Blutes erhöht, das während der Diastole durch den elastischen Rückzug der Aortenwurzel (Windkesselfunktion) vorwärts befördert wird.

Durch einen größeren Hub der Aortenwurzel wird der Rückzug der Aortenwurzel während der Diastole verstärkt und der Sog in beiden Herzkammern in der Diastole erhöht, wodurch die Vorhöfe besser entleert werden.

Durch einen größeren Hub der Aortenwurzel wird während der Systole die lange Achse der linken und der rechten Herzkammer stärker verkürzt und es kann mehr Blut aus beiden Herzkammern ausgeworfen werden.

Die Vergrößerung des Hubes der Aortenwurzel und damit verbunden des Herzskelettes bzw. der Herzbasis unterstützt die passive Pumpleistung der Aortenwurzel und auch der Pulmonaliswurzel in der Diastole sowie die aktive Pumpleistung der rechten und linken Herzkammer in der Systole. Durch die Verstärkung des enddiastolischen Soges in den Herzkammern wird die Vorlast beider Herzkammern erhöht und somit wird die Frank-Starling-Kurve nach rechts verschoben. Dies erniedrigt den enddiastolischen Druck in den Vorhöfen. Durch die Verstärkung der Windkesselfunktion sowohl in der Aortenwurzel als auch in der Pulmonaliswurzel wird die Nachlast beider Herzkammern verringert und damit das Auswurfvolumen beider Herzkammern vergrößert.

Eine Unterstützung des Aortenwurzelhubs kann sich positiv auf die diastolische Funktion des Herzens auswirken. Eine verminderte Elastizität und Rückstellkraft der Aorta vermindert den diastolischen Sog, die enddiastolische Füllung und damit die Vorlast und daraus resultierend die Leistungsfähigkeit des linken Ventrikels gemäß dem Frank-Starling-Mechanismus.

Eine Unterstützung der Dehnung der Aorta während der Systole erlaubt eine höhere Speicherung von Spannungsenergie in der Aorta, die in der Diastole zu einem verstärkten Rückzug der Aortenwurzel und dadurch einem erhöhten diastolischen Sog in der linken Herzkammer führt. Hierdurch kommt es zu einer verbesserten Entleerung der Vorhöfe und zu einer erhöhten Füllung und Vorlast der Ventrikel, was gemäß Frank-Starling-Mechanismus die Herzleistung verbessert.

Der Aortenwurzelhub wirkt positiv auf die Windkesselfunktion der Aortenwurzel und Aorta. Die Aortenwurzel ist ein Zylinder mit einem Durchmesser von durchschnittlich 3,5cm. Bei einem gesunden Menschen wird dieser Zylinder während der Systole um eine Länge von ca. 12 mm gestreckt. Das Volumen des Zylinders ist $V=\pi r^2 h=11{,}55$ ml. Dieses Volumen wird während der Diastole vorwärts gepumpt. Dies entspricht 16,5% eines durchschnittlichen Schlagvolumens von 70ml. Dabei ist noch nicht die radiale Dehnung und Windkesselfunktion der Aorta berücksichtigt, die bei einer verminderten Elastizität der Aortenwurzel ebenfalls vermindert ist. Eine Unterstützung des Aortenhubes führt zu einer zumindest teilweisen Wiederherstellung der Windkesselfunktion in Bezug auf die Verkürzung der Aorta in der Diastole.

Bei Patienten mit HFpEF sind die Dehnung und der Hub der versteiften Aortenwurzel reduziert. Bei diesen Patienten ist die Herzkraft normal oder kompensatorisch vergrößert. Die mangelnde Verkürzung der Herzkammern während der Systole kann nicht durch eine zusätzliche Reduktion des Durchmessers des Ventrikels kompensiert werden und hat eine Verminderung des Auswurfvolumens zur Folge. Eine Unterstützung des Aortenwurzelhubes mittels der erfindungsgemäßen Vorrichtung kann vorteilhaft zu einer Verkürzung der Längsachse des Herzens und damit zu einem verbesserten Auswurf des Herzens bei der Systole führen. Ein positiver Effekt der erfindungsgemäßen Vorrichtung auf die Muskelkontraktion der beiden Herzkammern und der beiden Vorhöfe kann ein Nebeneffekt des vergrößerten Aortenwurzelhubes sein.

Durch die Unterstützung der Längsdehnung der Aortenwurzel wird vorzugsweise das Hubvolumen des zuvor beschrieben Aortenwurzel-Zylinders vergrößert und das in der Diastole vorwärts gepumpte Blutvolumen erhöht. Dadurch wird der Druck in der Aortenwurzel zu Beginn der Systole entsprechend reduziert und die Nachlast des Herzens vermindert, wodurch sich das Schlagvolumen vergrößert.

In einigen Ausführungsformen ist die erfindungsgemäße Vorrichtung, insbesondere der erste Anker der Vorrichtung, nicht ausgebildet, um an der Mitralklappe (insbesondere deren Ring) oder um ausschließlich an der Mitralklappe (insbesondere deren Ring) befestigt zu werden. In einigen Ausführungsformen ist die Vorrichtung nicht ausgestaltet, um an einer Mitralklappenprothese angebracht zu werden. In einigen Ausführungsformen umfasst die Vorrichtung keine Mitralklappenprothese.

In manchen Ausführungsformen hat die erfindungsgemäße Vorrichtung, insbesondere der erste Anker der Vorrichtung, keine geschlossene Kreis- oder Ringform, auch nicht die Form eines Teilkreises (oder Teilrings), dessen Kreiswinkel über 180° hinausgeht. Er ist nicht schleifen- oder schlaufenförmig, insbesondere hat er nicht in Form einer schlaufenförmigen Anuloplastie, auch nicht einer teilweisen Anuloplastie (einer Mitralklappe).

In einigen Ausführungsformen hat die erfindungsgemäße Vorrichtung nicht die Form eines Kreises oder eines Teilkreises (oder eines Rings oder eines Teilrings), in dessen Haupterstreckungsebene eine oder mehrere Strukturen, z. B. Erweiterungen bzw. Erweiterungseinheiten, z. B. speichenförmig, angeordnet sind.

In manchen Ausführungsformen beginnt oder endet im inneren Bereich des Rings oder Teilrings (oder in einem von diesem umschlossenen Bereich) keine Führungseinrichtung. In manchen Ausführungsformen ist dort auch keine Führungseinrichtung mit einem Endabschnitt des Verbindungsstücks verbunden oder dafür vorgesehen.

In einigen Ausführungsformen weist die Vorrichtung keinen Abschnitt auf, der zum Aufliegen auf dem Rand einer Herzklappe, z. B. Mitralklappe oder Mitralklappenprothese, vorgesehen ist.

In manchen Ausführungsformen weist die Vorrichtung keine Struktur auf, die zum Verbleib im Herzen vorgesehen ist und sich dabei durch den Durchströmungsquerschnitt einer Herzklappe erstreckt.

In einigen Ausführungsformen ist die erfindungsgemäße Vorrichtung ausgestaltet, um zu mehr als 50 %, vorzugsweise mehr als 75 %, besonders vorzugsweise mehr als 85 % ihrer Länge innerhalb des (linken und/oder rechten) Ventrikels und/oder des (linken und/oder rechten) Vorhofs zu liegen.

Erfindungsgemäß ist die erfindungsgemäße Vorrichtung ausgestaltet, um ausschließlich an einer Seite des Herzens, insbesondere ausschließlich an der Herzspitze, die Herzwand zu penetrieren.

In einigen Ausführungsformen ist die Vorrichtung nicht ausgestaltet, um gleichzeitig an gegenüberliegenden Anteilen der Herzwand verankert zu werden.

In einigen Ausführungsformen ist die Vorrichtung ausgestaltet, um nach Einsetzen mit mindestens 30 %, vorzugsweise mindestens 50 %, besonders vorzugsweise mindestens 70 % ihrer Masse und/oder ihres Volumens außerhalb des linken Herzens, insbesondere außerhalb des linken Ventrikels angeordnet zu sein.

In einigen Ausführungsformen weist die Vorrichtung keinen Permanentmagneten und/oder keinen Elektromagneten auf.

Im Folgenden wird die erfindungsgemäße Vorrichtung anhand bevorzugter Ausführungsformen derselben unter Bezugnahme auf die angehängten Zeichnungen beschrieben. Die Erfindung ist jedoch nicht auf diese Ausführungsformen beschränkt. In den Zeichnungen gilt:

Fig. 1 zeigt eine Darstellung eines Herzens mit den vier Herzkammern, Herzbasis und Herzskelett;

Fig. 2 zeigt eine Darstellung der Pumpfunktion des Herzens;

Fig. 3 zeigt eine Darstellung der Aufhängung des Herzbeutels im Brustkorb;

Fig. 4 zeigt eine schematische Darstellung der Pumpfunktion der linken Herzkammer;

Fig. 5 zeigt eine Ausführungsform der erfindungsgemäßen Vorrichtung zur Unterstützung des Aortenhubes;

Fig. 6 zeigt eine schematische Darstellung der Funktion einer Ausführungsform der erfindungsgemäßen Vorrichtung zur Unterstützung des Aortenhubes;

Fig. 7 zeigt die erfindungsgemäße Vorrichtung in einer Ausführungsform;

Fig. 8 zeigt einen zweiten Anker einer Ausführungsform der erfindungsgemäßen Vorrichtung, der mit einer Rippe verbunden ist; und

Fig. 9 zeigt die erfindungsgemäße Vorrichtung im implantierten Zustand im Brustkorb mit einer Steuereinrichtung, wie hierin offenbart.

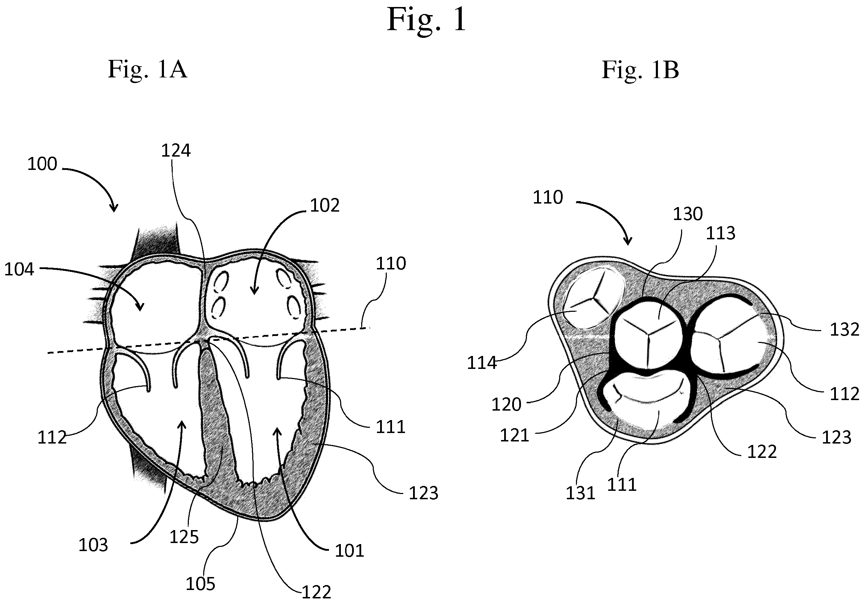

**Fig. 1** zeigt ein menschliches Herz 100 mit den vier Herzkammern (Ventrikel bzw. Atrium) 101, 102, 103, 104 und der Herzbasis 110 in einem Schnitt. In **Fig. 1A** sind der linke Ventrikel 101 (linke Herzkammer 101) und das linke Atrium 102 (linker Vorhof 102) sowie die dazwischenliegende Mitralklappe 111, der rechte Ventrikel 103 (rechte Herzkammer 103) und das rechte Atrium 104 (rechter Vorhof 104) mit der dazwischen liegenden Trikuspidalklappe 112 dargestellt. Zwischen den beiden Atria 102, 104 befindet sich das Vorhofseptum 124. Zwischen den beiden Ventrikeln 101 und 103 befindet sich das intraventrikuläre Septum 125.

In **Fig. 1B** ist die Herzbasis 110 dargestellt. Die Herzbasis 110 ist eine vergleichsweise ebene anatomische Struktur des Herzens 100, auf der sich die beiden atrioventrikulären Klappen, nämlich die Mitralklappe 111 und Trikuspidalklappe 112, sowie die beiden Taschenklappen, nämlich die Aortenklappe 113 und die Pulmonalklappe 114, befinden. Die Aortenklappe 113 wird von dem Aortenklappenring 130 umschlossen bzw. in dem Aortenklappenring 130 fixiert. Das Herzskelett 120 besteht aus knorpeligem Gewebe und ist die einzige steife Struktur des Herzens 100. Das Herzskelett 120 umfasst vollständig die Aortenwurzel 201 sowie die zentralen Anteile des Mitralklappenrings 131 und des Trikuspidalklappenrings 132. Die am kräftigsten ausgebildeten Anteile des Herzskelettes 120 sind das linke fibröse Trigon 121 und das rechte fibröse Trigon 122. Am rechten fibrösen Trigon 122 grenzen die Mitralklappe 111 und die Trikuspidalklappe 112 aneinander. Der Herzmuskel 123 des interventrikulären Septums 125 ist im Bereich des rechten fibrösen Trigons 122 zwischen Mitralklappe 111 und Trikuspidalklappe 112 mit dem Herzskelett 120 verbunden. An dieser Stelle führt die Kontraktion des Herzmuskels 123 des interventrikulären Septums 125 zu einem Zug am Herzskelett 120 und der damit verbundenen Aortenklappe 113 in Richtung Herzspitze.

Die Mitralklappe 111 und Trikuspidalklappe 112 schließen sich am Ende der Diastole und stellen sicher, dass das Blut bei Kontraktion der Ventrikel 101 und 103 nicht zurück in die Atria 102 und 104 fließt, sondern vorwärts und auf der rechten Seite in die Lungen und auf der linken Seite in den Körperkreislauf gepumpt wird. Die Aortenklappe 113 und die Pulmonalklappe 114 schließen sich am Ende der Systole und stellen sicher, dass nach erfolgter Kontraktion der beiden Ventrikel 101 und 103 das Blut nicht zurück in die Ventrikel 101 und 103 fließt, sondern auf der rechten Seite der diastolische Blutdruck in der Pulmonalarterie und der Lunge und auf der linken Seite der diastolische Druck in der Aorta und dem Körperkreislauf aufrechterhalten bleibt.

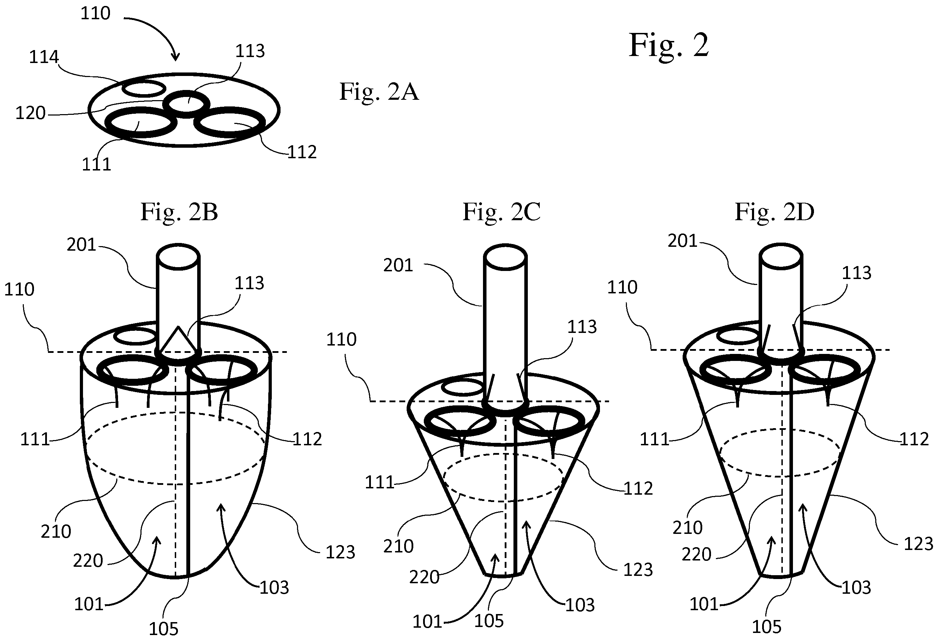

**Fig. 2** zeigt eine schematische Darstellung des Herzens. **Fig. 2A** zeigt die Herzbasis 110 schematisch. **Fig. 2B** zeigt das Herz 100 in der Diastole mit entspanntem Herzmuskel 123. Die Längsachse 220 ist hier im Kontraktionszyklus am längsten und der Umfang der Herzkammern 210 und der orthogonale Diameter am größten. Die beiden Herzkammern 101 und 103 sind gefüllt, die Mitralklappe 111 und Trikuspidalklappe 112 sind geöffnet, um den Bluteinstrom in die Herzkammern zu ermöglichen. Die Aortenklappe 113 ist geschlossen und verhindert so den Rückfluss von Blut aus dem Körperkreislauf in die linke Herzkammer 101 und erhält den diastolischen Blutdruck im Körper aufrecht. Die Aortenwurzel 201 ist am Ende der Diastole maximal zusammengezogen oder verkürzt.

**Fig. 2C** zeigt schematisch die Kontraktion des Herzens 100 in der Systole mit einer normal elastischen Aortenwurzel 201. Der Herzmuskel 123 ist kontrahiert, das Herz 100 hat den geringsten Umfang 210 und den geringsten orthogonalen Diameter im Herzzyklus. Die Herzspitze 105 verbleibt stationär, die Herzbasis 110 hat sich zur Herzspitze 105 verlagert und die Längsachse 220 ist am kürzesten. Die atrioventrikulären Klappen 111 und 112 sind geschlossen, um einen Rückfluss von Blut in die beiden Vorhöfe 102 und 104 zu verhindern. Die Aortenklappe 113 ist geöffnet, um den Auswurf von Blut in den Körperkreislauf zu ermöglichen. Die Aortenwurzel 201 ist maximal gestreckt.

**Fig. 2D** zeigt schematisch ebenfalls die Kontraktion des Herzens 100 in der Systole, jedoch bei steifer, nicht elastischer Aortenwurzel 201. Der Herzmuskel 123 ist kontrahiert, das Herz hat den geringsten Umfang 210 und den geringsten orthogonalen Diameter bezogen auf den Herzzyklus. Die Herzspitze 105 verbleibt stationär, die Lage der Herzbasis 110 hat sich gegenüber der Diastole nicht verändert, da die steife Aortenwurzel 201 nicht gedehnt werden kann und so die Herzbasis 110 nicht zur Herzspitze 105 hingezogen werden kann. Die Längsachse 220 ist ebenso lang wie beim entspannten Herzen 100 in der Diastole in **Fig. 2B.** Aufgrund der Muskelkontraktion ist jedoch der Umfang 210 und orthogonale Diameter am geringsten bezogen auf den Herzzyklus. Die atrioventrikulären Klappen 111 und 112 sind geschlossen, um einen Rückfluss von Blut in die beiden Vorhöfe 102 und 104 zu verhindern. Die Aortenklappe 113 ist geöffnet, um den Auswurf von Blut in den Körperkreislauf zu ermöglichen.

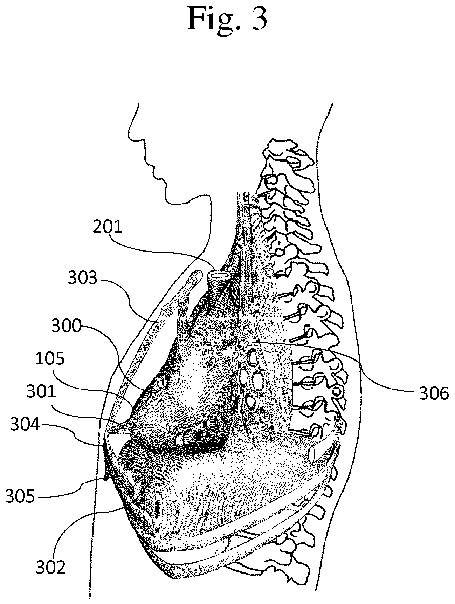

**Fig. 3** zeigt eine schematische Darstellung des Brustkorbes eines Menschen. Das Perikard 300 (Herzbeutel) liegt dem Zwerchfell 302 auf und ist zwischen der Aortenwurzel 201 mit dem Mediastinum 306 und dem sterno-perikardialen Ligament 301 aufgespannt. Das sterno-perikardiale Ligament 301 erstreckt sich vom Perikard 300 im Bereich der Herzspitze 105 zu einer Rippe 305 und zum Manubrium sterni am Ende des Sternums 303.

Bei Kontraktion des Herzens kann das Perikard 300 der Verringerung des Herzumfanges 210 während der Systole folgen. Da das geschlossene Perikard über das sterno-perikardiale Ligament 301 zwischen dem relativ unbeweglichen Sternum 303 und dem Mediastinum 306 aufgespannt ist, kann sich die Herzspitze 105 nicht vom Sternum 303 weg in Richtung zur Herzbasis 110 verlagern. Aus diesem Grund führt die Kontraktion des Herzens und Verkürzung der Längsachse 220 des Herzens zu einem Zug an der Herzbasis 110 und der elastischen Aortenwurzel 201, die auf diese Weise in der Systole gestreckt und die Herzbasis 101 mit der Aortenwurzel 201 in Richtung Herzspitze 105 gezogen wird.

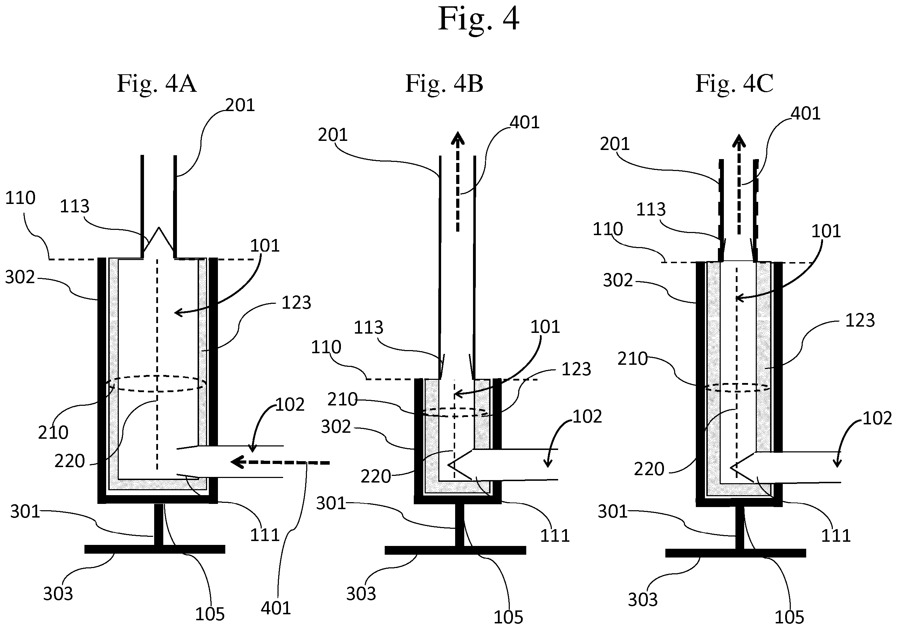

**Fig. 4** zeigt eine schematische Darstellung der Pumpfunktion eines Ventrikels 101 in Abhängigkeit von der Elastizität der Aortenwurzel 201.

**Fig. 4A** zeigt schematisch einen linken Ventrikel 101 in der Diastole. Der Herzmuskel 123 ist entspannt, die Längsachse 220 ist am längsten, der Umfang des Ventrikels 210 und der orthogonale Diameter sind am größten im Herzzyklus. Der Ventrikel 101 ist gefüllt und die Mitralklappe 111 ist geöffnet, um den Bluteinstrom 401 in den Ventrikel zu ermöglichen. Die Aortenklappe 113 ist geschlossen und verhindert so den Rückfluss von Blut aus dem Körperkreislauf in den linken Ventrikel und erhält den diastolischen Blutdruck im Körper aufrecht. Die Aortenwurzel 201 ist am Ende der Diastole maximal zusammengezogen.

**Fig. 4B** zeigt schematisch die Kontraktion des Herzens mit einer normal elastischen Aortenwurzel 201. Der Herzmuskel 123 ist kontrahiert, das Herz hat den geringsten Umfang 210 bzw. orthogonalen Diameter. Die Herzspitze 105 verbleibt stationär, da das Perikard 300 über das sterno-perikardiale Ligament 301 mit dem Sternum 303 verbunden ist. Aufgrund der Kontraktion des Herzmuskels 123 hat sich die Herzbasis 110 zur Herzspitze 105 verlagert und die Längsachse 220 ist am kürzesten im Herzzyklus. Die Mitralklappe 111 ist geschlossen, um den Rückfluss von Blut in das Atrium 102 verhindern. Die Aortenklappe 113 ist geöffnet, um den Auswurf von Blut 401 in den Körperkreislauf zu ermöglichen. Die Aortenwurzel 201 ist maximal gestreckt. Nach der Systole in **Fig. 4B** entspannt sich der Herzmuskel 123, der Umfang des Herzens 210 und sein orthogonaler Diameter nimmt wieder zu und die Aortenwurzel 201 zieht die Herzbasis 110 weg von der Herzspitze 105 **(Fig. 4A)**. Dadurch wird das in der Aortenwurzel 201 befindliche Blut während der Diastole in den Körperkreislauf gepumpt.

**Fig. 4C** zeigt schematisch ebenfalls die Kontraktion des linken Ventrikels 101, jedoch bei steifer, nicht elastischer Aortenwurzel 201. Der Herzmuskel 123 ist kontrahiert. Die Herzspitze 105 verbleibt stationär, da der Herzbeutel 300 über das sterno-perikardiale Ligament 301 mit dem Sternum 303 verbunden ist. Die Lage der Herzbasis 110 hat sich nicht verändert, da die steife Aortenwurzel 201 nicht gedehnt werden kann und so die Herzbasis nicht zur Herzspitze 105 hingezogen werden kann. Die Längsachse 220 ist ebenso lang wie bei dem entspannten Herzen in der Diastole in **Fig. 4A.** Aufgrund der Muskelkontraktion ist jedoch der Umfang 210 und der orthogonale Diameter am geringsten im Herzzyklus. Die Mitralklappe 111 ist geschlossen, um den Rückfluss von Blut in das linke Atrium 102 zu verhindern. Die Aortenklappe 113 ist geöffnet, um den Auswurf von Blut in den Körperkreislauf zu ermöglichen.

Nach der Systole **Fig. 4C** entspannt sich der Herzmuskel wieder und der Umfang 210 nimmt wieder zu **(Fig. 4A)**. Da die Herzbasis 110 stationär bleibt, findet im Vergleich zu der Situation mit elastischer Aortenwurzel **Fig. 4B** bei steifer Aortenwurzel 201 in **Fig. 4C** während der Diastole kein Vorwärtspumpen des Blutes in der Aortenwurzel 201 statt.

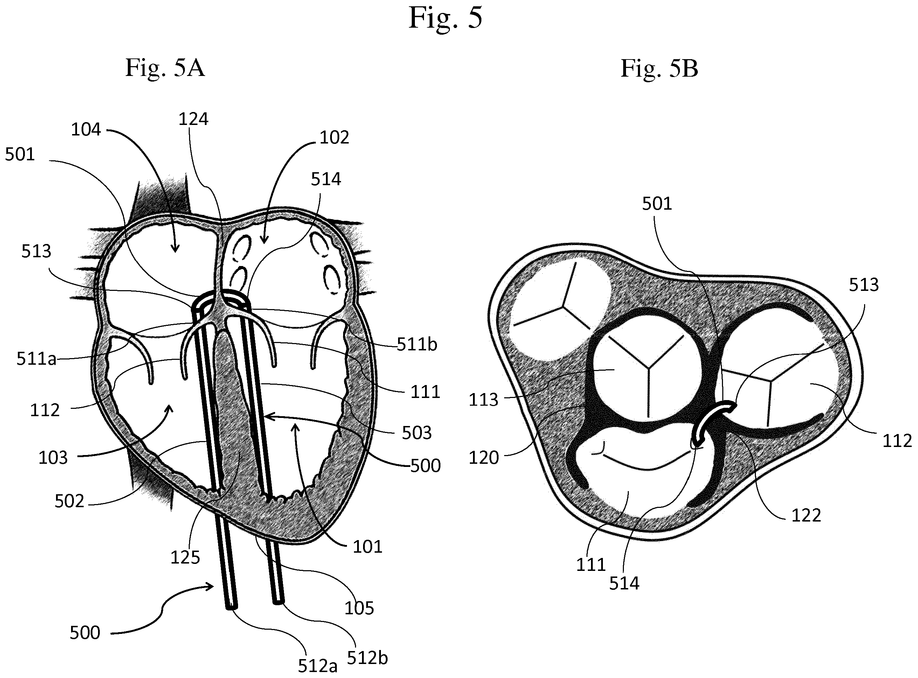

**Fig. 5** zeigt eine exemplarische Ausführungsform der erfindungsgemäßen Vorrichtung 500. Dabei zeigt **Fig. 5A** eine schematische Darstellung der Vorrichtung 500 in den Ventrikeln 101 und 103 sowie in den Atria 102 und 104. Die Vorrichtung 500 umfasst einen ersten Anker 501, der als Bügel oder als Verbindungsstück bezeichnet werden kann, und eine Führungseinrichtung 502, die als Hubantrieb bezeichnet werden kann, die optional im rechten Ventrikel 103 angeordnet ist, und eine Führungseinrichtung 503, die optional im linken Ventrikel 101 angeordnet ist.

In einigen Ausführungsformen beträgt die Länge der erfindungsgemäßen Vorrichtung 500 in der Diastole vorzugsweise zwischen 70 mm und 120 mm, besonders vorzugsweise zwischen 72 mm und 116 mm, in der Systole vorzugsweise zwischen 60 mm und 115 mm, besonders vorzugsweise zwischen 62 mm und 114 mm. Die Änderung der Länge der Vorrichtung 500 von Diastole zu Systole beträgt vorzugsweise minimal 0 mm und maximal 21 mm, besonders vorzugsweise minimal 10 mm und maximal 15 mm.

Der erste Anker 501 weist zwei Enden 513 und 514 auf. Ein erstes Ende 513 ist optional im implantierten Zustand am unteren Ende des rechten Vorhofes 104 in unmittelbarer Nähe zur Basis des septalen Segels der Trikuspidalklappe 112 angeordnet. Ein zweites Ende 514 des ersten Ankers 501 ist optional im implantierten Zustand am unteren Ende des linken Vorhofes 102 in unmittelbarer Nähe zur Basis des anterioren Segels der Mitralklappe 111 angeordnet. Der erste Anker 501 durchdringt optional das atriale Septum 124 am unteren Ende des atrialen Septums 124 und, wie in **Fig. 5B** dargestellt, oberhalb des rechten fibrösen Trigons 122, wo die Mitralklappe 111 und die Trikuspidalklappe 112 aneinandergrenzen.

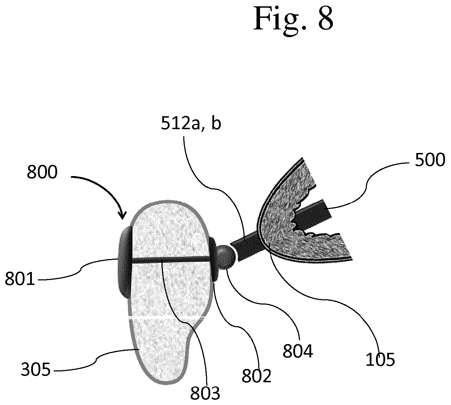

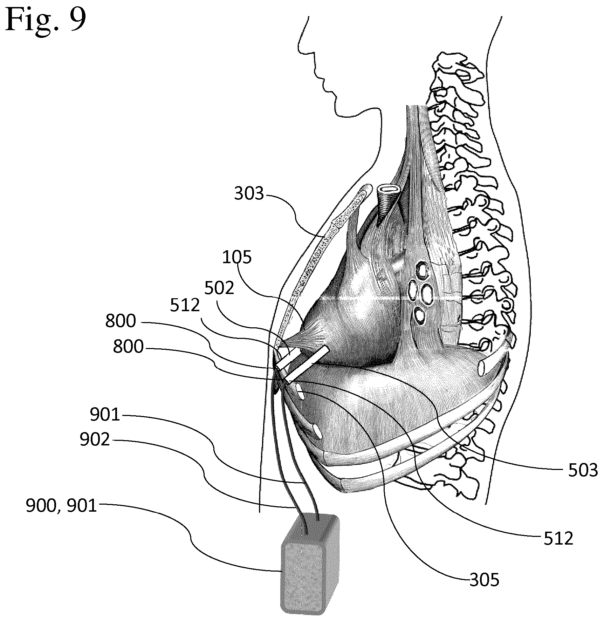

Der erste Anker 501 ist mit seinem ersten Ende 513 (Wenn hierin die Rede von einem Ende ist, kann dies optional als Endbereich verstanden werden. Diese beiden Begriffe können optional gegeneinander ausgetauscht werden.) im rechten Atrium 104 mit dem ersten Ende 511 der Führungseinrichtung 502 verbunden. Das erste Ende 511 der Führungseinrichtung 502 durchdringt das septale Segel der Trikuspidalklappe 112 unmittelbar an der Basis des Klappensegels. Die Führungseinrichtung 502 befindet sich in der rechten Herzkammer 103, direkt neben dem Ventrikelseptum 125 und durchdringt mit ihrem zweiten Ende 512a die Herzspitze 105. Das zweite Ende 512a der Führungseinrichtung 502 ist optional mit einer Rippe 305 verbunden (s. Fig. 8 und 9), alternativ kann das zweite Ende 512a z. B. direkt in der Herzspitze 105 oder mit dem sterno-perikardialen Ligament 301 verankert sein.

Der erste Anker 501 ist an seinem zweiten Ende 514 im linken Vorhof 102 mit dem ersten Ende 511b der zweiten Führungseinrichtung 503 verbunden. Vereinfachend wird im Folgenden die zweite Führungseinrichtung 503 auch als Führungseinrichtung bezeichnet. Das erste Ende 511b der Führungseinrichtung 503 hat das anteriore Segel der Mitralklappe 111 unmittelbar an der Basis des Klappensegels durchdrungen. Die zweite Führungseinrichtung 503 befindet sich in der linken Herzkammer 101, direkt neben dem Ventrikelseptum 125 und dringt mit seinem zweiten Ende 512b in die Herzspitze 105 hinein, vorzugsweise durch diese hindurch. Das zweite Ende 512b der zweiten Führungseinrichtung 503 ist optional mit einer Rippe 305 verbunden, alternativ kann das zweite Ende 512b direkt in der Herzspitze 105 oder am sterno-perikardialen Ligament 301 verankert sein.

Die erfindungsgemäße Vorrichtung 500 kann in einigen Ausführungsformen vollständig chirurgisch über eine offene Herzoperation implantiert werden. Alternativ kann die erfindungsgemäße Vorrichtung 500 in einigen Ausführungsformen über einen Kombinationseingriff mittels Katheter und chirurgischem Eingriff implantiert werden. Alternativ kann die erfindungsgemäße Vorrichtung 500 in einigen Ausführungsformen auch vollständig mittels Katheter eingebracht werden.

Die vollständige chirurgische Implantation erfolgt vorzugsweise über eine übliche Herzoperation. Alternativ kann die Implantation in einigen Ausführungsformen über einen Kombinationseingriff erfolgen, bei dem mittels Katheter der erste Anker 501 über die Vena cava und das rechte Atrium 104 in das atriale Septum eingebracht wird. Der Brustkorb wird über der Herzspitze 105 chirurgisch geöffnet und die beiden ersten Enden 511a und 511b der Führungseinrichtungen 502 und 503 werden über die Herzspitze 105 in das schlagende Herz entlang des Ventrikelseptums 125 eingeführt. Die Führungseinrichtungen 502 und 503 perforieren in diesem Fall von der Ventrikelseite aus die Trikuspidalklappe 112 und Mitralklappe 111. Die beiden ersten Enden 511a, 511b der Führungseinrichtungen 502, 503 werden dann unter Durchleuchtung am schlagenden Herzen mit dem entsprechenden Ende 513 bzw. 514 des ersten Ankers 501 verbunden. Die zweiten Enden 512a, 512b der Führungseinrichtungenen 502, 503 werden dann offen chirurgisch mit einer Rippe verbunden. Alternativ können die zweiten Enden 512a, 512b direkt in der Herzspitze 105 oder dem sterno-perikardialen Ligament 301 verankert werden.

Bei der vollständig kathetergestützten Implantation wird die Führungseinrichtung 502 über den rechten Vorhof 104 in das Herz eingeführt und das septale Segel der Trikuspidalklappe 112 mit dem zweiten Ende 512a der Führungseinrichtung 502 perforiert. Die Führungseinrichtung 502 wird bis in die Herzspitze 105 vorgeschoben und dort verankert. Alternativ kann die Herzspitze 105 mit dem zweiten Ende 512a der Führungseinrichtung 502 perforiert werden und dann im sterno-perikardialen Ligament 301 oder an der Rippe 305 verankert werden.

Die zweite Führungseinrichtung 503 wird über das rechte Atrium 104 in das Herz eingeführt und über eine Perforation des atrialen Septums 124 in das linke Atrium 102 vorgeschoben. Das anteriore Mitralklappensegel 111 wird mit dem zweiten Ende 512b der Führungseinrichtung 503 perforiert. Das zweite Ende 512b der Führungseinrichtung 503 wird bis in die Herzspitze 105 vorgeschoben und dort verankert. Alternativ kann die Herzspitze 105 mit dem zweiten Ende 512a der Führungseinrichtung 502 perforiert werden und dann im sterno-perikardialen Ligament 301 oder an der Rippe 305 verankert werden.

Der erste Anker 501 wird über das rechte Atrium 104 in das Herz eingeführt und das zweite Ende 514 über eine Perforation im Vorhofseptum 124 in das linke Atrium 102 eingebracht. Unter Durchleuchtung wird ein Verschlusssystem (nicht dargestellt) zwischen dem ersten Ende 514 des Verbindungsstückes 501 mit dem ersten Ende 511b der Führungseinrichtung 503 und zwischen dem zweiten Ende 513 des ersten Ankers 501 mit dem ersten Ende 511a der Führungseinrichtung 502 verbunden.

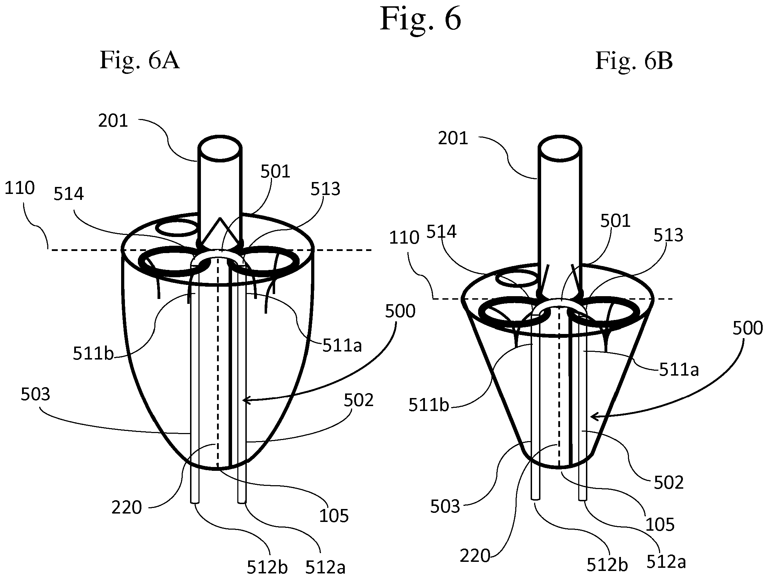

**Fig. 6** stellt schematisch die Funktion der erfindungsgemäßen Vorrichtung 500 nach erfolgter Implantation in einem Herzen mit einer steifen Aortenwurzel 201 dar, wie sie bereits in Fig. 4C beschrieben wurde. Es wird angenommen, dass die Herzkraft nicht ausreicht, um die steife Aorta 201 zu dehnen und die Herzbasis 110 hin zur Herzspitze 105 zu ziehen. In Fig. 4B wurde eine normal elastische Aorta 201 beschrieben, bei der die Herzbasis 110 zur Herzspitze 105 hingezogen werden kann.

**Fig. 6A** zeigt schematisch das Herz in der Diastole mit der implantierten erfindungsgemäßen Vorrichtung 500. Die Führungseinrichtungen 502 und 503 sind gestreckt, die lange Achse des Herzens 220 ist am längsten im Herzzyklus, die Herzbasis 110 ist am weitesten von der Herzspitze 105 entfernt und die Aortenwurzel 201 ist entsprechend kurz.

**Fig. 6B** zeigt das Herz am Ende der Systole schematisch. Es wird angenommen, dass die Herzkraft nicht ausreicht, um die steife Aortenwurzel 201 zu strecken. Die Führungseinrichtungen 502 und 503 haben sich aktiv verkürzt und die Herzbasis 110 und somit auch die steife Aortenwurzel 201 zu der Herzspitze 105 gezogen. Dadurch ist die Längsachse 220 am kürzesten im Herzzyklus und die steife Aortenwurzel 201 ist mittels der erfindungsgemäßen Vorrichtung 100 aktiv gestreckt.

Auf dem Weg zurück von der Systole in **Fig. 6B** hin zur Diastole in **Fig. 6A** zieht sich die Aortenwurzel 201 wieder zusammen. Dadurch bewegt sich die Herzbasis 110 weg von der Herzspitze 105 und die entsprechende Blutsäule in der Aortenwurzel 201 wird während der Diastole in den Körperkreislauf gepumpt.

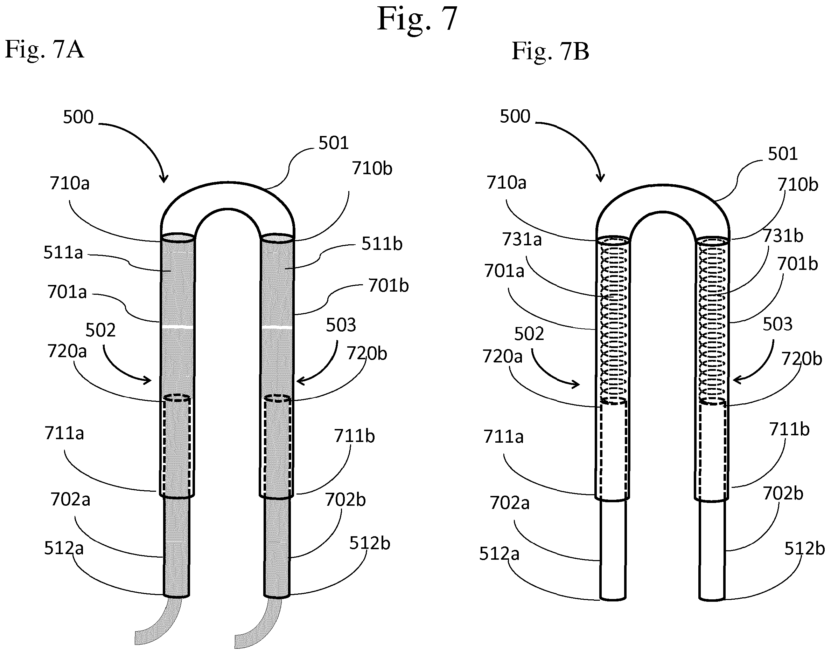

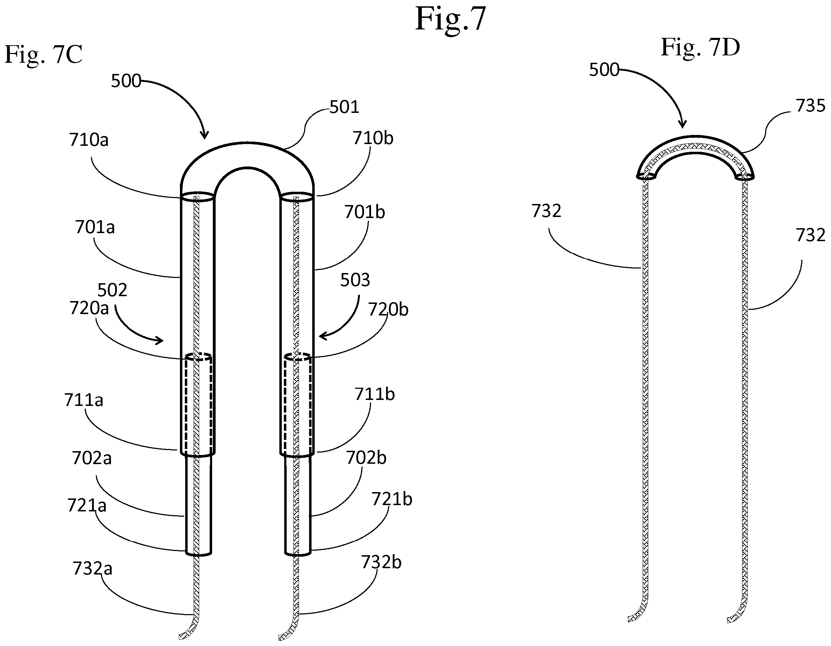

**Fig. 7** stellt schematisch vereinfacht dargestellte, alternative Ausführungsformen der erfindungsgemäßen Vorrichtung 500 dar. In **Fig. 7A** verkürzen sich die Führungseinrichtungen 502 und 503 durch hydraulische Kraft. Die Verkürzung erfolgt dadurch, dass die optional als Kolben ausgeführten Linearführungseinrichtungen 702a, 702b relativ zu den als Zylinder ausgeführten Linearführungsbuchsen 701a, 701b bewegt werden. Bei einer Verkürzung werden die Kolben in die Zylinder hineingeschoben, bei einer Verlängerung werden die Kolben aus den Zylindern herausgezogen. Die zweiten Enden 512a, 512b der Kolben 702a, 702b können beispielsweise an der Herzspitze 105, dem sterno-perikardialen Ligament 301 oder der Rippe 305 verankert werden. Durch Anlegen eines Unterdrucks am jeweils zweiten Ende 512a, 512b der Kolben 702a, 702b bewegen sich die ersten Enden 710a, 710b der Zylinder 701a, 701b hin zu den ersten Enden 720a, 720b der Kolben 702a, 702b. Daraus resultiert eine Verkürzung der Führungseinrichtungen 502 und 503 und der erste Anker 501 wird zur Herzspitze 105 gezogen.

In **Fig. 7B** verkürzen sich die Führungseinrichtungen 502 und 503 durch Spannkraft mittels jeweils einer Feder 731a, 731b. Das zweite Ende des Kolbens 512 ist an der Herzspitze 105, dem sterno-perikardialen Ligament 301 oder der Rippe 305 verankert. In den Führungseinrichtungen 502 und 503 befindet sich je eine Feder 731a, 731b, die das erste Ende des Zylinders 710a, 710b mit dem ersten Ende 720a, 720b des Kolbens 702a, 702b verbindet. Durch den Rückzug der Aortenwurzel 201 in der Diastole werden die Kolben 702a, 702b in den Zylindern 701a, 701b zum zweiten Ende des Zylinders 711a, 711b hin bewegt und die Federn 731a, 731b gespannt. Die Federn 731a, 731b können als Zugfedern bezeichnet werden. Während der Systole verkürzen sich die Federn 731a, 731b und die ersten Enden 710a, 710b der Zylinder 701a, 701b werden zum ersten Ende der Kolben 720a, 720b gezogen. Auf diese Art und Weise wird der erhöhte Kraftaufwand für die Dehnung der steifen Aorta 201 in der Systole durch die Spannung der Federn 731a, 731b zumindest teilweise ausgeglichen.

In **Fig. 7C** verkürzen sich die Führungseinrichtungen 502 und 503 mit Hilfe eines Seils 732. Die zweiten Enden 512a, 512b der Kolben 702a, 702b können an der Herzspitze 105, dem sterno-perikardialen Ligament 301 oder der Rippe 305 verankert werden. In den Zylindern 701a, 701b ist das Seil 732a, 732b jeweils am ersten Ende des Zylinders 710a, 710b befestigt. Das Ende des Seiles 732a, 732b wird an einem zweiten Anker (nicht dargestellt) in der Nähe der Herzspitze 105, dem sterno-perikardialen Ligament 301 oder der Rippe 305 umgeleitet.

Durch Zug an den Seilen 732a, 732b werden die Zylinder 701a, 701b über den jeweiligen Kolben 702a, 702b gezogen und das jeweilige erste Ende 710a, 710b der Zylinder 701a, 701b bewegt sich zu den ersten Enden 720a, 720b der Kolben 702a, 702b. Daraus resultiert eine Verkürzung der Führungseinrichtungen 502 und 503 und das Verbindungsstück 501 wird zur Herzspitze 105 gezogen.

In **Fig. 7D** ist eine erfindungsgemäße Vorrichtung 500 dargestellt, die lediglich aus einem als hohlem Verbindungsstück 735 ausgeführten ersten Anker 501 und einem Seil 732 besteht. Das Ende des Seiles 732 wird an einem zweiten Anker (nicht dargestellt) an der Herzspitze 105, am sterno-perikardialen Ligament 301 oder an der Rippe 305 umgeleitet. Durch Zug an einem Ende oder beiden Enden des Seiles 732 wird das Verbindungsstück 735 zur Herzspitze 105 gezogen.

Neben den beschriebenen Vorrichtungen 500 sind darüber hinaus weitere aktive Verkürzungsmechanismen denkbar und von der Erfindung umfasst, wie z.B. eine Verkürzung mittels magnetischer Kraft, mittels einem transplantierten Muskel, einem biotechnisch hergestellten Muskel oder einem künstlichen Muskel, die jeweils zur Erzeugung von Kraft genutzt werden können.

**Fig. 8** stellt einen zweiten Anker 800 an der Rippe 305 dar. Der Anker 800 wird mit einem Widerlager 801 an der Außenseite der Rippe 305, einem Widerlager 802 an der Innenseite der Rippe 305 und mit einer zentralen Verbindung 803 durch die Rippe 305 befestigt. Der zweite Anker 800 wird in örtlicher Nähe zur Herzspitze 105 in der Rippe 305 befestigt. Über ein Kugelgelenk 804 ist der zweite Anker 800 mit dem zweiten Ende 512a, 512b der Führungseinrichtung 502, 503 beweglich verbunden. Vorzugsweise sind beide Führungseinrichtungen 502 und 503 mit ihrem zweiten Ende 512a, 512b jeweils mit einem zweiten Anker 800 beweglich mit der Rippe 305 verbunden.

Neben dem beschriebenen zweiten Anker 800 an der Rippe sind weitere Ausformungen und Ankerformen vorstellbar (z.B. auch lediglich die Verwendung eines chirurgischen Fadens), die ebenfalls von der vorliegenden Erfindung umfasst sind.

**Fig. 9** stellt einen Brustkorb mit einer implantierten erfindungsgemäßen Vorrichtung 500 dar. Die beiden zweiten Enden 512a, 512b der Führungseinrichtungen 502 und 503 sind mit je einem zweiten Anker 800 mit der Rippe 305 verbunden. Über die Verbindungen 902 ist die jeweilige Führungseinrichtung 502 und 503 mit der Steuereinheit 901 und/oder mit der Energiequelle 900 verbunden. Je nach Ausführung wird die benötigte Hubkraft der Führungseinrichtung über die Verbindungen 902 als hydraulische Kraft, Zugkraft, Rotationskraft, elektromagnetische Kraft oder in anderer Form übertragen. Die Steuereinheit 901 kann über eine EKG-Messung oder eine Druckmessung mit der natürlichen Herztätigkeit synchronisiert werden. Die Steuereinheit 901 kann einen elektrischen Motor, einen Linearmotor, Getriebe, Pumpen oder andere Vorrichtungen enthalten, die zur Erzeugung und Übertragung der benötigten Kraft über die Verbindungen 902 notwendig sind. Die Steuereinheit 901 kann Energiequellen wie Batterien oder Akkus enthalten, die über ein externes Ladegerät aufgeladen werden können. Auch andere Energiequellen wie z.B. Nuklearenergie sind vorstellbar, um die notwendige Energie zur Verfügung zu stellen, und sind von der vorliegenden Erfindung umfasst.

## Bezugszeichen

| | |
|---|---|
| 100 | Herz |
| 101 | linker Ventrikel, linke Herzkammer |
| 102 | linkes Atrium, linker Vorhof |
| 103 | rechter Ventrikel, rechte Herzkammer |
| 104 | rechtes Atrium, rechter Vorhof |
| 105 | Herzspitze |
| 110 | Herzbasis |
| 111 | Mitralklappe |
| 112 | Trikuspidalklappe |
| 113 | Aortenklappe |
| 114 | Pulmonalklappe |
| 120 | Herzskelett |
| 121 | linkes fibröses Trigon |
| 122 | rechtes fibröses Trigon |
| 123 | Herzmuskel |
| 124 | atriales Septum, Vorhofseptum |
| 125 | interventrikuläres Septum, Herzkammerscheidewand |
| 130 | Aortenklappenring |
| 131 | Mitralklappenring |
| 132 | Trikuspidalklappenring |
| 201 | Aortenwurzel |
| 210 | Umfang der Herzkammern in der Diastole, orthogonaler Diameter |
| 220 | Längsachse der Herzkammern |
| 300 | Perikard, Herzbeutel |
| 301 | sterno-perikardiales Ligament |
| 302 | Diaphragma, Zwerchfell |
| 303 | Sternum |
| 304 | Processus xiphoideus |
| 305 | Rippe |
| 306 | Mediastinum, Mittelfellraum |
| 401 | Bluteinstrom |
| 500 | Vorrichtung zur Unterstützung der Herzaktion |
| 501 | erster Anker; Bügel; Verbindungsstück zwischen den beiden Führungseinrichtungen |
| 502 | Führungseinrichtung; Hubantrieb |
| 503 | Führungseinrichtung; zweite Führungseinrichtung; Hubantrieb |
| 511a,b | erstes Ende der Führungseinrichtung |
| 512a,b | zweites Ende der Führungseinrichtung |
| 513 | erstes Ende des Verbindungsstücks 501 im rechten Vorhof |
| 514 | zweites Ende des Verbindungsstücks 501 im linken Vorhof |
| 701a,b | Zylinder; Linearführungsbuchse |
| 702a,b | Kolben; Linearführungseinrichtung |
| 710a,b | erstes Ende des Zylinders |
| 711a,b | zweites Ende des Zylinders |
| 720a,b | erstes Ende des Kolbens |
| 721a,b | zweites Ende des Kolbens |
| 731a,b | Feder |
| 732 | Zugeinrichtung; Seil |
| 732a,b | Zugeinrichtung; Seil |
| 735 | erster Anker; Verbindungsstück zwischen den Vorhöfen |
| 800 | zweiter Anker; optional an der Rippe angeordnet |
| 801 | Widerlager an der Außenseite der Rippe |
| 802 | Widerlager an der Innenseite der Rippe |
| 803 | Verbindung vom Widerlager an der Außenseite der Rippe mit dem Widerlager an der Innenseite der Rippe |
| 804 | Kugelgelenk |
| 900 | Energiequelle |
| 901 | Steuereinheit |
| 902 | Verbindung von Steuereinheit mit Führungseinrichtung |

## Patentansprüche

1.    Vorrichtung (500) zur Unterstützung der Herzaktion, vorzugsweise durch Verlagerung der Herzbasis (110) und/oder der Aortenwurzel (201), umfassend wenigstens einen ersten Anker (501) und eine Zug- oder Führungseinrichtung (502, 503, 732, 732a, 732b) zum Bewegen des ersten Ankers (501), wobei der erste Anker (501) zur Implantation in oder an der Herzbasis (110), dem Herzskelett (120) oder der Aortenwurzel (201) vorgesehen und ausgestaltet ist, wobei der erste Anker (501) ein Bügel ist oder aufweist, und wobei die Vorrichtung (500) ausgestaltet ist, um ausschließlich auf einer Seite des Herzens die Herzwand zu penetrieren.

2. Vorrichtung (500) nach Anspruch 1, wobei die Zug- oder Führungseinrichtung (502, 503, 732, 732a, 732b) eine Linearführungseinrichtung (702a, 702b) ist oder aufweist.

3. Vorrichtung (500) nach Anspruch 2, wobei der erste Anker (501) einen V- oder U-förmigen Abschnitt aufweist und der erste Anker (501) mit wenigstens einer, vorzugsweise zwei, Linearführungsbuchse(n) (701a, 701b) mit einem, vorzugsweise beiden, Ende(n) des ersten Ankers (501) verbunden ist bzw. sind, wobei die Linearführungsbuchse(n) (701a, 701b) zum Führen der Linearführungseinrichtung(en) (702a, 702b) ausgestaltet ist.

4. Vorrichtung (500) nach einem der Ansprüche 2 bis 3, wobei die Linearführungseinrichtung (702a, 702b) ein Kolben (702a, 702b) ist oder einen solchen aufweist.

5. Vorrichtung (500) nach einem der Ansprüche 2 bis 4, wobei die Linearführungseinrichtung (702a, 702b) mechanisch, hydraulisch, pneumatisch, elektrisch oder magnetisch bewegbar ist.

6. Vorrichtung (500) nach einem der Ansprüche 3 bis 5, wobei eine Zugfeder (731a, 731b) in der Linearführungsbuchse (701a, 701b) zwischen der Linearführungseinrichtung (702a, 702b) und dem ersten Anker (501) angeordnet ist.

7. Vorrichtung (500) nach einem der Ansprüche 3 bis 6, wobei die Verbindung zwischen dem ersten Anker (501) und der Linearführungsbuchse (701a, 701b) eine Steckverbindung, eine Klemmverbindung, ein Bajonettverschluss oder eine andere Verbindung ist oder aufweist.

8. Vorrichtung (500) nach Anspruch 1, wobei die Zug- oder Führungseinrichtung (502, 503, 732, 732a, 732b) ein längliches, biegeweiches und zugfestes Element, insbesondere ein Seil (732) oder ein Riemen, ist oder aufweist.

9. Vorrichtung (500) nach einem der vorhergehenden Ansprüche, wobei die Vorrichtung (500) einen zweiten Anker (800) zur Implantation in oder an der Herzspitze (105), einem Ligament, einer Rippe und/oder einem Brustbein aufweist.

10. Vorrichtung (500) nach einem der vorhergehenden Ansprüche, wobei die Vorrichtung (500) aus einem Metall, einem Kunststoff und/oder einem Verbundwerkstoff hergestellt ist oder eine solches Material aufweist, wobei das Material vorzugsweise biokompatibel ist.

11. Einführsystem aufweisend eine Vorrichtung (500) nach einem der Ansprüche 1 bis 10 oder Abschnitte hiervon, insbesondere die Führungseinrichtung (502, 503, 732, 732a, 732b), nicht aber den ersten Anker (501), wobei das Einführsystem einen Einführungskatheter, einen Führungskatheter, einen Führungsdraht und/oder mindestens einen Abgabekatheter umfasst.

12. Kit, aufweisend entweder eine Vorrichtung (500) nach einem der Ansprüche 1 bis 10 und eine Steuereinrichtung (901), umfassend ein mechanisches, hydraulisches, pneumatisches, elektrisches oder magnetisches Antriebssystem zum Antreiben einer Zug- oder Führungseinrichtung (502, 503, 732, 732a, 732b) der Vorrichtung nach einem der Ansprüche 1 bis 10, und/oder aufweisend ein Einführsystem nach Anspruch 11.

**Claims**

1. A device (500) for supporting heart action, preferably by shifting the heart base (110) and/or the aortic root (201), comprising at least a first anchor (501) and a pulling or guiding device (502, 503, 732, 732a, 732b) for moving the first anchor (501), wherein the first anchor (501) is provided and designed for implantation in or on the heart base (110), the heart skeleton (120) or the aortic root (201), wherein the first anchor (501) is or comprises a bracket, and wherein the device (500) is designed to penetrate the heart wall exclusively on one side of the heart.

2. The device (500) according to claim 1, wherein the pulling or guiding device (502, 503, 732, 732a, 732b) is, or comprises, a linear guiding device (702a, 702b).

3. The device (500) according to claim 2, wherein the first anchor (501) comprises a V- or U-shaped section and the first anchor (501) is connected with at least one, preferably two, linear guiding bush (es) (701a, 701b) at one, preferably both, end(s) of the first anchor (501), wherein the linear guiding bush(es) (701a, 701b) is/are designed to guide the linear guiding device(s) (702a, 702b).

4. The device (500) according to any one of the claims 2 to 3, wherein the linear guiding device (702a, 702b) is a piston (702a, 702b) or comprises such a piston.

**5.** The device (500) according to any one of the claims 2 to 4, wherein the linear guiding device (702a, 702b) is mechanically, hydraulically, pneumatically, electrically, or magnetically movable.

**6.** The device (500) according to any one of the claims 3 to 5, wherein a tension spring (731a, 731b) is arranged in the linear guiding bush(es) (701a, 701b) between the linear guiding device (702a, 702b) and the first anchor (501).

**7.** The device (500) according to any one of the claims 3 to 6, wherein the connection between the first anchor (501) and the linear guiding bush (es) (701a, 701b) is or comprises a plug connection, a clamp connection, a bayonet lock or another connection.

**8.** The device (500) according to claim 1, wherein the pulling or guiding device (502, 503, 732, 732a, 732b) is or comprises an elongated, flexural-soft, and tensile-resistant element, in particular a rope (732) or a belt.

**9.** The device (500) according to any one of the preceding claims, wherein the device (500) comprises a second anchor (800) for implantation in or on the heart apex (105), a ligament, a rib and/or a sternum.

**10.** The device (500) according to any one of the preceding claims, wherein the device (500) is made of a metal, a plastic and/or a composite material or comprises such material, wherein the material is preferably biocompatible.

**11.** An insertion system comprising a device (500) according to any one of the claims 1 to 10 or sections thereof, in particular the guiding device (502, 503, 732, 732a, 732b), but not the first anchor (501), wherein the insertion system comprises an insertion catheter, a guiding catheter, a guide wire and/or at least one delivery catheter.

**12.** A kit comprising either a device (500) according to any one of the claims 1 to 10 and a control device (901) comprising a mechanical, hydraulic, pneumatic, electrical, or magnetic drive system for driving the pulling or guiding device (502, 503, 732, 732a, 732b) of the device according to any one of claims 1 to 10, and/or comprising an insertion system according to claim 11.

**Revendications**

**1.** Un dispositif (500) destiné à soutenir l'activité cardiaque, de préférence par déplacement de la base du cœur (110) et/ou de la racine aortique (201), comprenant au moins un premier ancrage (501) ainsi qu'un dispositif de traction ou de guidage (502, 503, 732, 732a, 732b) pour déplacer le premier ancrage (501), où le premier ancrage (110) est prévu et conçu pour une implantation dans ou sur la base du cœur (110), le squelette du cœur (120) ou la racine aortique (201), où le premier ancrage (501) est ou comporte un étrier, et où le dispositif (500) est conçu pour pénétrer la paroi cardiaque exclusivement d'un côté du cœur.

**2.** Le dispositif (500) selon la première revendication, où le dispositif de traction ou de guidage (502, 503, 732, 732a, 732b) est, ou comporte, un dispositif de guidage linéaire (702a, 702b).

**3.** Le dispositif (500) selon la revendication 2, où le premier ancrage (501) présente une partie en forme de V ou de U et le premier ancrage (501) est relié à au moins une, de préférence deux, douille(s) de guidage linaire (701a, 701b) à une, de préférence aux deux, extrémité(s) du premier ancrage (501), où la/les douilles(s) de guidage linaire (701a, 701b) est/sont conçue(s) pour guider le(s) dispositif(s) de guidage linéaire (702a, 702b).

**4.** Le dispositif (500) selon l'une quelconque des revendications 2 à 3, où le dispositif de guidage linéaire (702a, 702b) est un piston (702a, 702b), ou comporte un tel piston.

**5.** Le dispositif (500) selon l'une quelconque des revendications 2 à 4, où le dispositif de guidage (702a, 702b) est mobile mécaniquement, hydrauliquement, pneumatiquement, électriquement ou magnétiquement.

**6.** Le dispositif (500) selon l'une quelconque des revendications 3 à 5, où un ressort de tension (731a, 731b) est disposé dans la/les douille (s) de guidage linéaire (701a, 701b) entre le dispositif de guidage linéaire (702a, 702b) et le premier ancrage (501).

**7.** Le dispositif (500) selon l'une quelconque des revendications 3 à 6, où la liaison entre le premier ancrage (501) et la/les douille (s) de guidage linéaire (701a, 701b) est, ou comporte, une liaison par enfichage, une liaison par serrage, une fermeture à baïonnette ou une autre liaison.

**8.** Le dispositif (500) selon la première revendication, où le dispositif de traction ou de guidage (502, 503, 732, 732a, 732b) est, ou comporte, un élément allongé, souple en flexion et résistant à la traction, notamment une corde (732) ou une courroie.

**9.** Le dispositif (500) selon l'une quelconque des revendications précédentes, où le dispositif (500) comprend un second ancrage (800) pour une implantation dans ou sur l'apex cardiaque (105), un ligament, une côte et/ou un sternum.

10. Le dispositif (500) selon l'une quelconque des revendications précédentes, où le dispositif (500) est fabriqué à partir d'un métal, d'un plastique et/ou d'un matériau composite ou comprend un tel matériau, où le matériau est de préférence biocompatible.

11. Un système d'introduction comprenant un dispositif (500) selon l'une quelconque des revendications 1 à 10 ou des parties de celles-ci, notamment le dispositif de guidage (502, 503, 732, 732a, 732b), mais pas le premier ancrage (501), où le système d'insertion comprend un cathéter d'insertion, un cathéter de guidage, un fil guide et/ou au moins un cathéter de délivrance.

12. Un ensemble comprenant un dispositif (500) selon l'une quelconque des revendications 1 à 10 et un dispositif de commande (901) comprenant un système d'entraînement mécanique, hydraulique, pneumatique, électrique ou magnétique pour entraîner le dispositif de traction ou de guidage (502, 503, 732, 732a, 732b) du dispositif selon la l'une quelconque des revendications 1 à 10, et/ou comprenant un système d'insertion selon la revendication 11.

# Fig. 1

## Fig. 1A

## Fig. 1B

Fig. 2

Fig. 2A

Fig. 2B

Fig. 2C

Fig. 2D

EP 3 856 277 B1

Fig. 3

Fig. 4

Fig. 4A   Fig. 4B   Fig. 4C

Fig. 5

Fig. 5A

Fig. 5B

# Fig. 6

## Fig. 6A

## Fig. 6B

EP 3 856 277 B1

# Fig. 7

## Fig. 7A

500
501
710b
710a
511b
511a
701b
701a
502
503
720a
720b
711a
711b
702a
702b
512a
512b

## Fig. 7B

500
501
710b
710a
731b
731a
701b
701a
502
503
720a
720b
711a
711b
702a
702b
512a
512b

EP 3 856 277 B1

Fig. 7

Fig. 7C

Fig. 7D

Fig. 8

Fig. 9

EP 3 856 277 B1

**IN DER BESCHREIBUNG AUFGEFÜHRTE DOKUMENTE**

**In der Beschreibung aufgeführte Patentdokumente**

- WO 2011119100 A1
- WO 2016102561 A1
- US 20060178550 A1
- WO 2016133203 A1